# EUROPEAN PATENT APPLICATION

(11) **EP 4 749 651 A1**
(43) Date of publication of application: **27.05.2026**
(21) Application number: 25217259.8
(22) Date of filing: 20.11.2025
(51) Int. Cl.: G16H 40/60

(54) **COLLECTION OF USER CHOICES AND RESULTING OUTCOMES FROM SURGERIES TO PROVIDE WEIGHTED SUGGESTIONS FOR FUTURE DECISIONS**

(30) Priority: 20.11.2024 US 202418954221
(71) Applicant: Cilag GmbH International, 6300 Zug (CH)
(72) Inventor: COWPERTHWAIT, Matthew David, Cincinnati, 45242 (US); ADAMS, Shane R., Cincinnati, 45242 (US); MUELLER, Karl W., Cincinnati, 45242 (US); ROSS, Nicholas James, Cincinnati, 45242 (US); HARRIS, Jason L, Cincinnati, 45242 (US); SHELTON IV, Frederick E., Cincinnati, 45242 (US); RUOFF, Joshua R., Cincinnati, 45242 (US); MADHOGARHIA, Rachel Garima, Cincinnati, 45242 (US); HONTZ, Jesse, Cincinnati, 45242 (US); TIMM, Richard W., Cincinnati, 45242 (US); WARD, Andréas N., Cincinnati, 45242 (US); JONES, Shannon L., Cincinnati, 45242 (US)
(74) Representative: Carpmaels & Ransford LLP

(57) **Abstract**

Systems, methods, and/or instrumentalities disclosed herein may collect user choices and/or resulting outcomes from surgeries to provide weighted suggestions for future decisions. A system may be configured to receive an user input indicating a selection of a procedure and/or a tactical domain target. The system may be configured to determine a tactical domain data for the procedure. The tactical domain data may include one or more relationships associated with a primary surgical element, a secondary surgical element, the parameter of the patient, and/or the tactical domain target. The system may be configured to generate a recommendation based on the tactical domain data. The recommendation may include an indication of an optimized control loop for the primary surgical element. The system may be configured to send the recommendation to the primary surgical element to adjust an output characteristic associated with the primary surgical element to achieve the tactical domain target.

## Description

### CROSS-REFERENCE TO RELATED APPLICATIONS

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
- U.S. Patent Application No. 18/954,186, filed November 20, 2024, entitled METHOD FOR MULTI-SYSTEM INTERACTION.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Systems, methods, and/or instrumentalities disclosed herein may collect user choices and/or resulting outcomes from surgeries to provide weighted suggestions for future decisions. A system may include a processor. The system may be configured to receive an user input indicating a selection of a procedure from a plurality of procedures and/or a selection of a tactical domain target. The procedure and/or the tactical domain target may be associated with a parameter of a patient. The system may be configured to filter, based on the selection of the procedure, a plurality of surgical elements to obtain a primary surgical element and/or a secondary surgical element associated with the procedure. The primary surgical element may include a plurality of primary control loops associated with an output characteristic of the primary surgical element. The secondary surgical element may include a plurality of secondary control loops associated with an output characteristic of the secondary surgical element. The system may be configured to determine a tactical domain data for the procedure. The tactical domain data may include one or more relationships associated with the primary surgical element, the secondary surgical element, the parameter of the patient, and/or the tactical domain target. The system may be configured to receive a primary control data from the primary surgical element based on a primary control loop from the plurality of primary control loops. The primary control data may include the output characteristic associated with the primary surgical element. The system may be configured to receive a secondary control data from the secondary surgical element based on a secondary control loop from the plurality of secondary control loops. The secondary control data may include the output characteristic associated with the secondary surgical element. The system may be configured to generate a recommendation based on the tactical domain data, the primary control data, and/or the secondary control data. The recommendation may include an indication of an optimized control loop for the primary surgical element during the procedure. The optimized control loop may adjust the output characteristic associated with the primary surgical element to achieve the tactical domain target. The system may be configured to send the recommendation to the primary surgical element. The system may be configured to cause the primary surgical element to adjust the output characteristic associated with the primary surgical element based on the optimized control loop. The primary surgical element may adjust the output characteristic during the procedure to achieve the tactical domain target.

One or more of features may be included. In examples, the parameter of the patient may include at least one of oxygen saturation, blood pressure, respiratory rate, blood sugar, heart rate, a core body temperature and/or a hydration state. The tactical domain target may be a core body temperature setpoint of the patient, the primary surgical element may be a heating blanket, the secondary surgical element may be a ventilator, the output characteristic associated with the primary surgical element may be a heating coil of the heating blanket, the output characteristic associated with the secondary surgical element may be a heating coil to adjust the temperature of air flowing through the ventilator. The recommendation may include the indication of the optimized control loop to be used by the primary surgical element to control the heating coil of the heating blanket to meet the core body temperature setpoint.

The system may be configured to obtain historical data associated with the procedure. The historical data may include historical control data for the primary surgical element and/or for the secondary surgical element. The system may be configured to determine, for the procedure, conflict data. The conflict data may include a determination of a conflict associated with the primary surgical element and/or the secondary surgical element and/or a request for a second user input indicating whether the determination of the conflict occurred during the procedure.

The system may be configured to generate the recommendation further based on a machine learning (ML) model. The ML model may be trained using training data including one or more training data items. A training data item of the one or more training data items may include at least one indication of the historical data associated with the procedure and/or conflict data.

The system may be configured to determine the tactical domain data further based on an ML model. The ML model may infer the one or more relationships associated with the primary surgical element, the secondary surgical element, the parameter of the patient, and/or the tactical domain target. The system may be configured to generate the recommendation based on an ML model associated with the tactical domain data, the primary control data, and/or the secondary control data. The one or more relationships associated with the primary surgical element, the secondary surgical element, the parameter of the patient, and/or the tactical domain target may be determined based on a look-up-table.

A method may include receiving an user input indicating a selection of a procedure from a plurality of procedures, and/or a selection of a tactical domain target. The procedure and/or the tactical domain target may be associated with a parameter of a patient. The method may include filtering, based on the selection of the procedure, a plurality of surgical elements to obtain a primary surgical element and/or a secondary surgical element associated with the procedure. The primary surgical element may include a plurality of primary control loops associated with an output characteristic of the primary surgical element. The secondary surgical element may include a plurality of secondary control loops associated with an output characteristic of the secondary surgical element. The method may include determining a tactical domain data for the procedure. The tactical domain data may include one or more relationships associated with the primary surgical element, the secondary surgical element, the parameter of the patient, and/or the tactical domain target. The method may include receiving a primary control data from the primary surgical element based on a primary control loop from the plurality of primary control loops. The primary control data may include the output characteristic associated with the primary surgical element. The method may include receiving a secondary control data from the secondary surgical element based on a secondary control loop from the plurality of secondary control loops. The secondary control data may include the output characteristic associated with the secondary surgical element. The method may include generating a recommendation based on the tactical domain data, the primary control data, and/or the secondary control data. The recommendation may include an indication of an optimized control loop for the primary surgical element during the procedure. The optimized control loop may adjust the output characteristic associated with the primary surgical element to achieve the tactical domain target. The method may include sending the recommendation to the primary surgical element. The method may include causing the primary surgical element to adjust the output characteristic associated with the primary surgical element based on the optimized control loop. The primary surgical element may adjust the output characteristic during the procedure to achieve the tactical domain target.

One or more of features may be included. In examples, the parameter of the patient may include at least one of oxygen saturation, blood pressure, respiratory rate, blood sugar, heart rate, a core body temperature and/or a hydration state. The tactical domain target may be a core body temperature setpoint of the patient, the primary surgical element may be a heating blanket, the secondary surgical element may be a ventilator, the output characteristic associated with the primary surgical element may be a heating coil of the heating blanket, and/or the output characteristic associated with the secondary surgical element may be a heating coil to adjust the temperature of air flowing through the ventilator. The recommendation may include the indication of the optimized control loop to be used by the primary surgical element to control the heating coil of the heating blanket to meet the core body temperature setpoint.

The method may include obtaining historical data associated with the procedure. The historical data may include historical control data for the primary surgical element and/or for the secondary surgical element. The method may include determining, for the procedure, conflict data. The conflict data may include a determination of a conflict associated with the primary surgical element and/or the secondary surgical element. Conflict data may include a request for a second user input indicating whether the determination of the conflict occurred during the procedure.

The method may include generating the recommendation further based on a machine learning (ML) model. The ML model may be trained using training data including one or more training data items, each training data item of the one or more training data items may include at least one indication of the historical data associated with the procedure and/or conflict data.

The method may include determining the tactical domain data further based on an ML model. The ML model may infer the one or more relationships associated with the primary surgical element, the secondary surgical element, the parameter of the patient, and/or the tactical domain target. The method may include generating the recommendation based on an ML model associated with the tactical domain data, the primary control data, and/or the secondary control data

A system may include a processor. The system may be configured to receive an user input indicating a selection of a procedure from a plurality of procedures, and/or a selection of a tactical domain target. The procedure and/or the tactical domain target may be associated with a parameter of a patient. The system may be configured to determine a tactical domain data for the procedure. The tactical domain data may include one or more relationships associated with a primary surgical element, a secondary surgical element, the parameter of the patient, and/or the tactical domain target. The system may be configured to generate a recommendation based on the tactical domain data. The recommendation may include an indication of an optimized control loop for the primary surgical element. The optimized control loop may adjust an output characteristic associated with the primary surgical element to achieve the tactical domain target. The system may be configured to send the recommendation to the primary surgical element.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 shows an example surgical instrument.
FIG. 6 is a block diagram for applying machine learning to improve artificial intelligence algorithms and/or iterations of learning for artificial intelligence algorithms.
FIG. 7 is an example operational environment in which a surgical computing system may receive control data to determine an optimized control loop for a surgical element.
FIG. 8 is an illustration of an example user interface indicating one or more conflicts.
FIG. 9 is a flow chart of an example optimized control loop selection routine performed by a surgical computing system.

### DETAILED DESCRIPTION

Operating rooms are becoming more sophisticated with the introduction of smart devices (e.g., interchangeably referred to herein as "surgical elements"). Smart devices may be used and/or adjusted by a health care personnel (HCP) during a procedure. Smart devices may include one or more advanced capabilities to significantly enhance the precision, safety, and efficiency of a procedure (e.g., a surgical procedure, diagnostic procedure, therapeutic procedure, preventative procedure and/or the like), while reducing the risk of complications to patients. Examples of smart devices may include robotic surgical systems, navigation systems, smart imaging systems, endoscopic and/or laparoscopic systems, energy scalpels, anesthesia machines, patient monitoring systems (pulse oximeters, blood pressure monitors, EKG monitors, EEG monitors, and/or the like), energy devices (e.g., electrosurgical units, laser surgery systems, and/or the like), infusion pumps, and/or the like.

Smart devices may include multiple control loops, each optimized for one or more steps of a procedure. The control loops may modify one or more characteristics associated with a smart device. For example, a control loop may modify the flexibility and/or rigidity of a smart device, energy settings of a smart device (e.g., the power level, waveform, and/or coagulation mode of a cutting tool), irrigation settings of a smart device (e.g., flow rates and/or temperature control), safety settings of a smart device, and/or the like. An HCP may manually set one or more control loops for a portion of a procedure (e.g., a specific step, a sub-step, an operation, an act, a surgical step, a surgical workflow, an intraoperative procedure, an operative sequence and/or the like) based on an HCP preference, desired performance, industry standards, and/or the like.

In some examples, one or more smart devices may be connected to a network. Smart devices may send and/or receive information (e.g., data) to and/or from a surgical computing system (e.g., interchangeably referred to herein as a "surgical hub"), another smart device, a third party (e.g., a database) and/or the like. Although one or more smart devices may be connected to a network, the network and/or smart devices may not coordinate with one another to achieve optimized results during a procedure, such as selecting an optimized control loop during a portion of a procedure.

For example, a patient's body temperature may be regulated using one or more smart devices during an endoscopic procedure. To avoid hypothermia, an HCP may analyze a patient's body temperature and manually adjusts the settings of one or more smart devices (e.g., warming blankets, warming pads, the temperature controller (thermostat) of the operating room, forced-air warming devices, warmed IV fluids, body temperature monitors, irrigation warming devices, anesthetic devices, and/or the like). As operating conditions change during the endoscopic procedure, repetitive monitoring and/or manual adjustments of one or more smart devices may unnecessarily increase an HCP's workload, affecting the efficiency, safety, and overall success of the endoscopic procedure.

As a typical example, during an endoscopic procedure an anesthetic device may sedate a patient's body, resulting in a drop in core body temperature. An HCP must analyze, and manually adjust a setpoint associated with multiple smart devices to compensate for the patient's loss of heat. If the patient's body temperature decreases during a critical portion of an endoscopic procedure (e.g., such as during insertion) the HCP may not have the time to adequately assess and/or determine the one or more relationships between smart devices to make an optimal adjustment to precisely control the patient's body temperature. Additionally, an HCP may not adequately understand how an adjustment to a first smart device may affect the performance of additional smart devices during a portion of the procedure. Consistent, manual adjustments by the HCP may result in an increased workload for the HCP, ultimately leading to fatigue, errors, and/or prolonged procedure times that potentially compromise patient safety and surgical outcomes.

To resolve issues described herein, systems and methods may include a surgical computing system (referred to herein as a "system"). A system may determine one or more relationships associated with one or more surgical elements for a procedure. The system may receive real-time data generated by smart devices during a procedure (e.g., operational data), real-time data associated with a patient's physiological parameters, and/or a tactical domain target during a procedure, and in response, recommend an optimal control loop for the one or more smart devices, determine a new tactical domain target, recommend a smart device to be used by an HCP during a portion of a procedure, and/or identify or resolve conflicts to reduce the workload of an HCP. The system may determine one our more outputs (e.g., a tactical domain target, an optimized control loop, a conflict, and/or the like) based on an artificial intelligence/machine learning model (ML model). The ML model may be trained with training data including historical data generated by one or more smart devices during past procedures, historical data associated with a patient's physiological parameters (e.g., biomarkers), and/or historical data associated with the outcomes of one or more past procedures.

A more detailed understanding may be had from the following description, given by way of example in conjunction with the accompanying drawings.

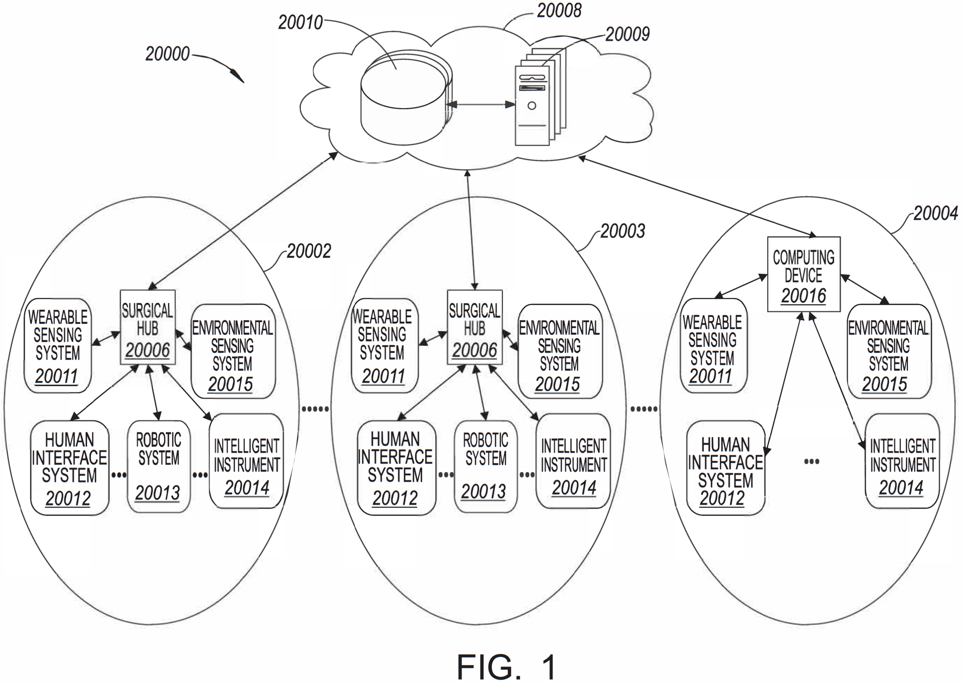

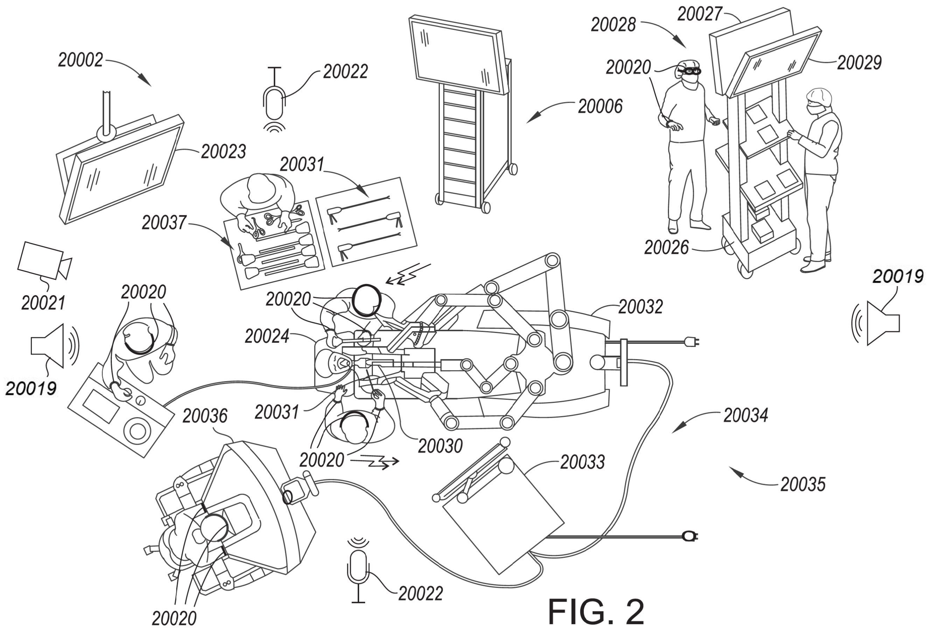

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more HCP sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a HCP. An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

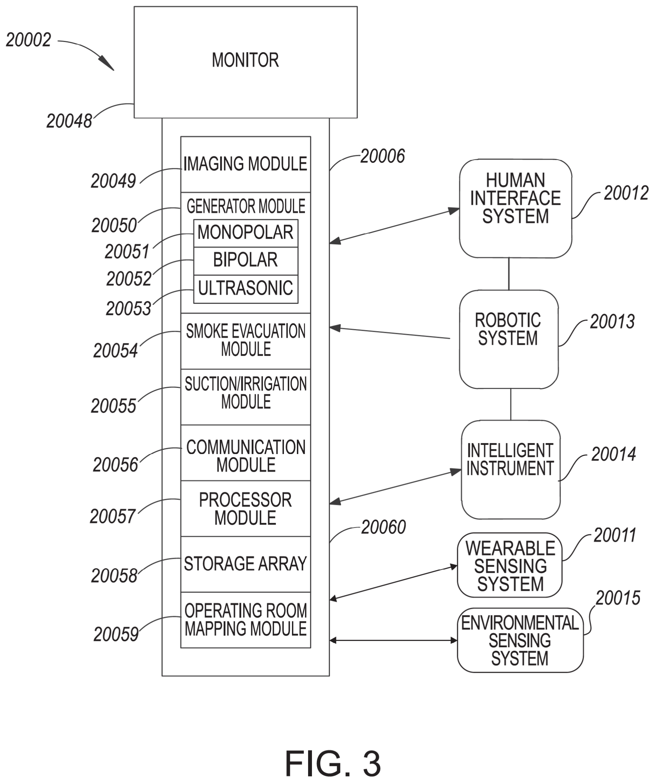

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

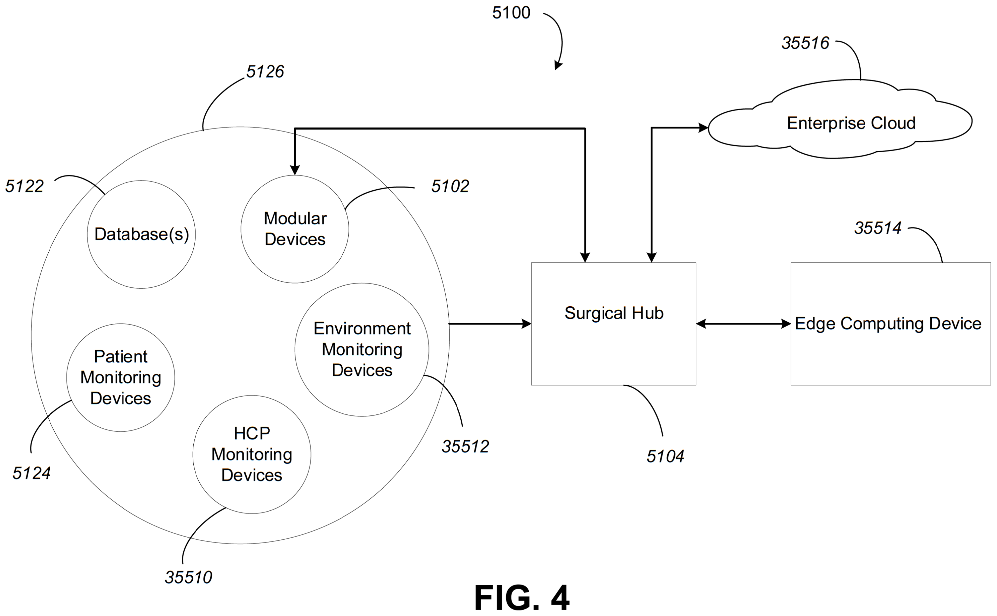

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

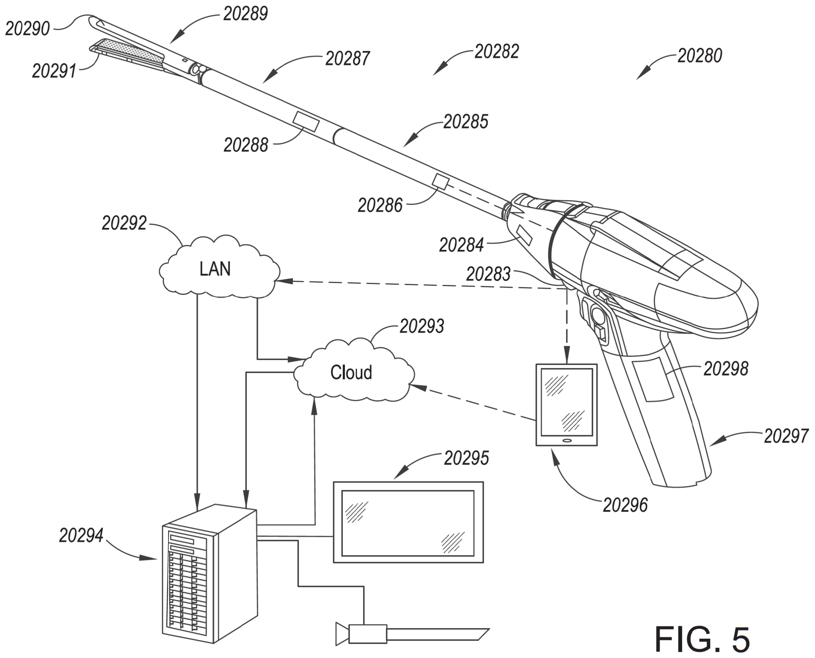

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

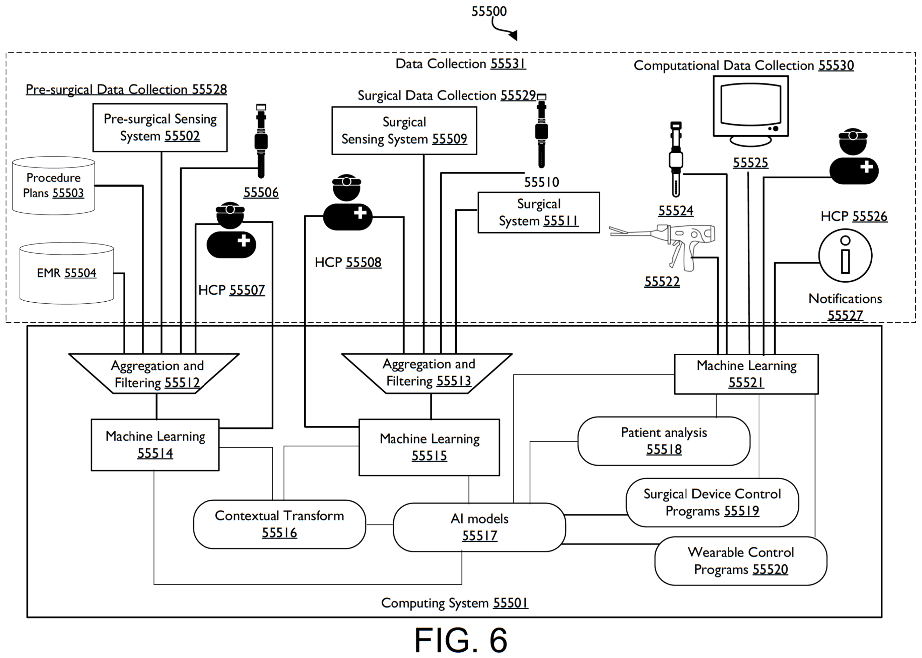

FIG. 6 depicts a block diagram for applying machine learning to improve artificial intelligence algorithms and/or iterations of learning for artificial intelligence algorithms. The computing system 55500 (e.g., surgical computing system and/or surgical computing hub) may include computing hardware including a processor, memory, input/output sub-systems, and the like. The processor may be configured (via application specific hardware, software, firmware, or the like) to transform received data and to derive contextualized for output. For example, the processor may include a microprocessor, a microcontroller, a FPGA, and an application-specific integrated circuit (ASIC), a system-on-a-chip (SOIC), a digital signal processing (DSP) platform, a real-time computing system, or the like. For example, processor may be configured to implement computing functions and/or modules as disclosed herein. For example, the processor may be configured for aggregation and/or filtering 55512, aggregation and/or filtering 55513, machine learning 55514, machine learning 55515, machine learning 55521, contextual transform 55516 (e.g., including real-time intra-operative processing), artificial intelligence models 55517, patient analysis 55518, surgical device control programs 55519, wearable control programs 55520, and the like.

The computing system 55500 may be any device suitable for processing sensor, health record data, user input, and the like, to transform the data and derive computational data for output. The computational output may include a sensor measurement. The computational output may include contextual information or a context, for example, which may include additional information relevant to the present understanding and/or interpretation of the sensor measurement. For example, the context may include pre-surgery and/or pre-therapy baselines. For example, the context may include situational awareness of incorrectly connected and/or incorrectly operating surgical and/or sensing systems. For example, the context may include adjustments to products, surgical plans, and/or margins.

The computing system 55500 may be incorporated into the system 55500 with any method suitable for implementation of the functionality disclosed herein. For example, the computing system 55500 may be incorporated as a stand-alone computing system. For example, the computing system may be incorporated into a surgical hub. For example, the computing system 55500 may be incorporated into a sensing system itself (e.g., sensing both pre-surgical and surgical data and providing contextualized data as an output). For example, the computing system 55500 may be incorporated into a surgical device itself (receiving both pre-surgical and surgical data and providing contextualized data, computational data, and/or alerts as an output).

A data collection, such as data collection 55531 may be provided. Machine learning may use the data collection, such as data collection 55531. Data collection 55531 may be used by machine learning to train, verify, create, and/or determine an ML model.

Data collection 55531 may include one or more data sources. For example, data collection 55531 may include pre-surgical data collection 55529, surgical data collection 55529, and computational data collection 55530. Data collection 55531 may include one or more biomarkers. The one or more biomarkers may come from one or more computing systems, surgical sensing systems, wearable devices, displays, surgical instruments, surgical devices, sensor systems, devices, and the like. The data collection 55531 may include electronic medical records for a patient, data for a patient, data for other patients, data regarding past procedures, data regarding research for procedures, medical data, instructions from a health care provider, plans for a surgery, and the like.

Data collection may include data from a number of different sources. For example, the sources may include procedure plans database 55503, EMR 55504, pre-surgical sensing system 55502, wearable device 55506, data from health care provider 55507, surgical sensing system 55509, HCP 55508, wearable device 55510, surgical system 55511, wearable device 55524, surgical device 55522, human-interface device 55525, data from HCP 55526, and data related to notifications 55527.

Data collection 55531 may include pre-surgical data collection 55528. Pre-surgical data collection 55528 may include data from one or more data sources. Pre-surgical data collection 55528 may include data that is related to a patient that may be recorded prior to a surgery. Pre-surgical data collection 55528 may include one or more biomarkers they may have been recorded for a patient prior to a surgery. For example, a heart rate and blood glucose level for a patient may be recorded for a patient prior to a surgery.

Pre-surgical data collection 55528 may include data from pre-surgical sensing system 55502. Pre-surgical sensing system 55502 may include any configuration of hardware and software devices suitable for sensing and presenting patient parameters and/or biomarkers that may be relevant before, during, or after a surgical procedure. Such a pre-surgical sensing system 55502 may include any of the sensing and monitoring systems disclosed herein, including uncontrolled patient monitoring systems, controlled patient monitoring systems, and the like. For example, pre-surgical sensing system 55502 may include a wearable patient sensor system. The pre-surgical sensing system 55502 may provide data suitable for establishing baselines of patient biomarkers for use in contextual determination during and/or after surgery. The pre-surgical sensing system 55502 may provide data suitable for establishing baselines of patient biomarkers for use in making predications and/or creating computational data.

Pre-surgical data collection 55528 may include data from wearable device 55506. Wearable device 55506 may include any configuration of hardware and software devices suitable for sensing and presenting patient parameters and/or biomarkers that may be relevant before, during, or after a surgical procedure. Such systems may be used by the patient for any amount of time prior to surgery, inside and outside of a medical facility. To illustrate, via an uncontrolled patient monitoring system, the patient may use a wearable heart-related sensor at home for four weeks prior to a surgical procedure. And/or, via a controlled patient monitoring system, a HCP may monitor the same and/or analogous biomarkers using facility equipment during time the patient is prepped immediately before the surgical procedure. For example, the wearable device 55506 may provide data suitable for establishing baselines of patient biomarkers for use in contextual determination and/or for use in creating computational data.

Pre-surgical data collection 55528 may include procedure plans 55503. Procedure plans 55503 may include any data source relevant to a health procedure (e.g., relevant to a health procedure in view of a particular patient and/or facility). Procedure plan 55503 may include structured data indicative of the desired end result, the surgical tactics to be employed, the operation logistics, and the like. Procedure plan 55503 may include an accounting of the equipment to be used and/or the techniques to be used. Procedure plan 55503 may include an order. Procedure plan 55503 may include a timeline. The structured data may include defined fields and/or data tags associated corresponding values. The structured data may include codes associated with surgical steps.

Pre-surgical data collection 55528 may include EMR 55504. EMR 55504 may include any data source relevant to a patient in view of a health procedure, such a surgical procedure. EMR 55504 may include information such as allergies and/or adverse drug reactions, chronic diseases, family medical history, illnesses and/or hospitalizations, imaging data, laboratory test results, medications and dosing, prescription record, records of surgeries and other procedures, vaccinations, observations of daily living, information collected by pre-surgical sensing system 55502, information collected by wearable device 55506, and the like.

Pre-surgical data collection 55528 may include data from a pre-surgical healthcare provider, such as HCP 55507. Data from HCP 55507 may include any data relevant to a pre-surgical sensing system 55502, a patient record, a procedure plan, and the like. Data from HCP 55507 may include data that may be relevant to an operation, configuration, and/or management of a computing system, such as computing system 55500. For example, data from HCP 55507 include feedback that may be provided to a machine learning module, such a machine learning module 55514. The data from HCP 55507 may include manually entering data that may not be received directly for a relevant source (such as manually entering a manually taken biomarker reading, for example).

Data collection 55531 may include surgical data collection 55529. Surgical data collection 55529 may include data from one or more data sources. Surgical data collection 55529 may include data that may be related to a patient that may be recorded during a surgery. Surgical data collection 55529 may include one or more biomarkers they may have been recorded for a patient during a surgery. For example, a heart rate and blood glucose level for a patient may be recorded for a patient during a surgery.

Surgical data collection 55529 may include one or more data sources. Surgical data collection 55529 may include data from surgical sensing system 55509, HCP 55508, surgical system 55511, and wearable device 55510.

Surgical data collection 55529 may include data from surgical sensing system 55509. Surgical sensing system 55509 may include any configuration of hardware and software devices suitable for sensing and presenting parameters patient biomarkers that may be relevant during a surgical procedure. Surgical sensing system 55509 may include the sensing and monitoring systems disclosed herein, including controlled patient monitoring systems, surgeon monitoring systems, environmental sensing systems, and the like.

Surgical data collection 55529 may include data from surgical sensing system 55509. Surgical sensing system 55509 may include any configuration of hardware and software devices suitable for sensing and presenting parameters patient biomarkers that may be relevant during a surgical procedure. Surgical sensing system 55509 may include one or more of the sensing and monitoring systems disclosed herein, including controlled patient monitoring systems, surgeon monitoring systems, environmental sensing systems, and the like.

Surgical data collection 55529 may include data from wearable device 55510. Wearable device 55510 may include any configuration of hardware and software devices suitable for sensing and presenting patent parameters and/or biomarkers that may be relevant before, during, or after a surgical procedure. Such systems may be used by the patient for any amount of time prior to surgery, inside and outside of a medical facility. To illustrate, via an uncontrolled patient monitoring system, the patient may use a wearable heart-related sensor at during a surgical procedure. And/or, via a controlled patient monitoring system, a healthcare provider may monitor the same and/or analogous biomarkers using facility equipment during time of the surgical procedure. For example, the wearable device 55510 may provide data suitable for use in a contextual determination and/or in a creation of computational data.

Surgical system 55511 may include any surgical equipment suitable for providing operative data regarding its configuration, use, and/or present condition and/or status, for example. Surgical system 55511 may include equipment in the surgical theater. Surgical system 55511 may include any equipment employed in the surgical theater as described herein. The surgical system 55511 may include surgical fixtures of a general nature, such as a surgical table, lighting, anesthesia equipment, robotic systems, and/or life-support equipment. Surgical system 55511 may include surgical fixtures that may be related to the procedure at-hand, such as imaging devices, surgical staplers, energy devices, endocutter clamps, and the like. For example, surgical system 55511 may include be one or more of a powered stapler, a powered stapler generator, an energy device, an energy device generator, an in-operating-room imaging system, a smoke evacuator, a suction-irrigation device, an insufflation system, or the like.

The surgical system 55511 may include at least one of a surgical instrument, surgical visualization system, monitor, sound system, energy devices, a wearable, and the like. For example, the surgical system may include a surgical hub. For example, the surgical system may include a surgical stapler. For example, the surgical system may include an endocutter, for example. Data from the surgical instrument may include surgical instrument parameters. The surgical instrument parameters may include surgical instrument power, for example. Data from the surgical visualization system may include location of surgical instruments in relation to a patient surgical site and/or organ. For example, data may include the distance between a surgical stapler and a close vital organ.

Surgical data collection 55529 may include data from a surgical HCP, such as HCP 55508. Data from HCP 55508 may include any data relevant to a surgical sensing system, a wearable device, a surgical system, a machine learning, a patient analysis, a surgical device control program, a wearable control program, a contextual transform, an artificial intelligence model, and the like. For example, HCP 55508 may provide data that may be associated with surgical system 55511, wearable device 55510, surgical sensing system 55509, machine learning 55515, and the like. For example, the HCP 55508 may provide data that may trigger an interaction with the contextual transform 55516 and/or machine learning 55515. The data from HCP 55508 may include manually entering data not received directly for any relevant source (such as manually entering a manually taken biomarker reading, for example).

The data received from the pre-surgical data sources, such as pre-surgical data collection 55528, may be subject to aggregation and/or filtering 55512. Aggregation and/or filtering 55512 may perform preprocessing on data received from pre-surgical data collection 55528. The data received from the surgical data sources, such as surgical data collection 55529, may be subject to aggregation and/or filtering 55513. Aggregation and/or filtering 55513 may perform per-processing on data received from surgical data collection 55529. Aggregation and/or filtering 55512 and aggregation and/or filtering 55513 may be used to prepare and format the data for use by computing system 55500. For example, aggregation and/or filtering 55512 and aggregation and/or filtering 55513 may prepare data to be processed by machine learning 55514, machine learning 55515, machine learning 55521, contextual transform 55516, artificial intelligence models 55517, surgical device control programs 55519, and wearable control programs 55520.

Processing the data received from the pre-surgical data collection 55528 by aggregation and/or filtering 55512 may include filtering (e.g., to select sensor data from the stream of data from pre-surgical sensing system 55502). Aggregation and/or filtering 55512 may use filtering to help reject noise in data from pre-surgical data collection 55502. Aggregation and/or filtering 55512 may use a method to establish a baseline for a biomarker from pre-surgical data collection 55528. Aggregation and/or filtering 55512 may perform time mapping on data from pre-surgical data collection 55528 (e.g., to place received values from different sources in alignment with each other in regard to time). Time mapping may aid in correlation and ratio analysis, which may occur in contextual transform 55516.

Aggregation and/or filtering 55512 may translate data from pre-surgical data collection 55528. The translation of data may include coordinating formats, coordinating data types, translating from one format to another format, translating from one data type to another data type, accounting for a difference between a data source data format, and accounting for a data type expected by another module, such as machine learning 55514. Translating may include translating the data into a format suitable for machine learning, for artificial intelligence models, for patient analysis, for use by a surgical device control program, and/or for use by a wearable control program. Data from the pre-surgical data collection 55528 may be translated into a notification for display, such as display on a human-interface device 55525. Data from pre-surgical data collection 55528 may be translated into a setting for the surgical device 55522. Data from surgical data collection 55529 may be translated into data that may be included and/or used for notifications 55527.

Processing the data received from surgical data collection 55529 by aggregation and/or filtering 55513 may include filtering (e.g., to select sensor data from the stream of data from surgical system 55511). Aggregation and/or filtering 55513 may use a method to establish a baseline for a biomarker from surgical data collection 55529. Aggregation and/or filtering 55513 may use filtering to help reject noise in data from surgical data collection 55529. Aggregation and/or filtering 55513 may perform time mapping on data from surgical data collection 55529 (e.g., to place received values from different sources in alignment with each other in regard to time). Time mapping may aid in correlation and ratio analysis, which may occur in contextual transform 55516.

Aggregation and/or filtering 55513 may translate data from surgical data collection 55529. The translation of data may include coordinating formats, coordinating data types, translating from one format to another format, translating from one data type to another data type, accounting for a difference between a data source data format, and accounting for a data type expected by another module, such as machine learning 55515. Translating may include translating the data into a format suitable for machine learning, for artificial intelligence models, for patient analysis, for use by a surgical device control program, and/or for use by a wearable control program. Data from the surgical data collection 55529 may be translated into a notification for display, such as display on a human-interface device 55525. Data from surgical data collection 55529 may be translated into a setting for the surgical device 55522. Data from surgical data collection 55529 may be translated into data that may be included and/or used for notifications 55527.

Contextual transform 55516 may operate to provide a context for data, such as pre-surgical data collection 55528 and/or surgical data collection 55529. For example, contextual transform 55516 may transform data into contextualized surgical data, which may be included in computational data collection 55530. To illustrate, as an input the contextual transform may receive surgical data that includes, for example, a measurement time, a sensor system identifier, and a sensor value. Contextual transform 55516 may output contextualized surgical data. Contextual transform 55516 may output data that may be modified and/or enhanced by machine learning 55514, machine learning 55515, machine learning 55521, patient analysis 55518, surgical device control programs 55519, wearable control programs 55520, and artificial intelligence models 55517.

Contextual transform 55516 may determine and/or store data that may be related to each other. Contextual transform 55516 may determine how data may be related to each other. For example, contextual transform 55516 may determine that data from surgical data collection 55529 may be related to data from pre-surgical data collection 55529. Contextual transform 55516 may determine a context for the data. Context, for example, additional information relevant to the present understanding and/or interpretation of the sensor measurement.

Computational data collection 55530 may include data that may be generated, created, determined, and/or computed by computing system 55500. For example, computational data collection 55530 may include models output from machine learning, data generated by machine learning, biomarkers processed by computing system 55500, augmented data, predictive probabilities, firmware, firmware updates, parameters for surgical devices, surgical device control program, updates to surgical device control programs, wearable control programs, parameters for wearable devices, parameters for controlling surgical devices, electronic medical records, contextual data, contextual surgical data, notifications, requests for feedback, messages to healthcare providers, and the like.

Computational data collection 55530 may include context, for example, additional information relevant to the present understanding and/or interpretation of the sensor measurement. For example, the context may include pre-surgery and/or pre-therapy baselines. For example, the context may include situational awareness of incorrectly connected and/or incorrectly operating surgical and/or sensing systems. For example, the context may include adjustments to products, surgical plans, and/or margins. Computational data collection 55530 may include data sent to or received from surgical device 55522, wearable device 55524, human-interface device 55525, HCP 55526, and notifications 55527. Computational data collection 55530 may be created, modified, received by and/or sent by machine learning 55514, machine learning 55515, machine learning 55521, contextual transform 55516, artificial intelligence models 55517, patient analysis 55518, surgical device control programs 55519, wearable control programs 55520, and/or any combination thereof.

Computational data collection 55530 may include data that provides a context. The context may include additional information that may place a biomarker into a specific context for the healthcare providers. For example, computational data collection 55530 may include instructions and/or information about a baseline value for a sensor value, an alert of a deviation, relevant information from the patient's record, relevant information to a procedural element of the surgery, surgical device settings, and/or any information the healthcare provider might find relevant to have at the moment of the sensor's measurement itself. The context may be determined by machine learning, such as by machine learning 55514, machine learning 55515, and/or machine learning 55521. Computational data collection 55530 may include one or more data tags. The data tags may include logging data (indicating that that a specific transform or other processing has occurred).

Computational data collection 55530 may include data that may be provided by HCP 55526. For example, HCP 55526 may provide feedback regarding data provided by machine learning 55521. Computational data collection 55530 may include data that may be sent to HCP 55526. For example, HCP 55526 may receive data provided by machine learning 55521. Data from HCP 55526 may include any data relevant to a surgical sensing system, a wearable device, a surgical system, a machine learning, a patient analysis, a surgical device control program, a wearable control program, a contextual transform, an artificial intelligence model, and the like. For example, HCP 55526 may provide data that may be associated with surgical device 55522, wearable device 55524, a patent, human-interface device 55525, notifications 55527, computing system 55500, and/or any combination thereof. For example, the HCP 55526 may provide data that may trigger an interaction with the contextual transform 55516 and/or machine learning 55521. The data from HCP 55526 may include manually entering data not received directly for any relevant source (such as manually entering a manually taken biomarker reading, for example).

Human-interface device 55525 may include any device suitable for producing a perceptible representation of computational data, such as computational data collection 55530. The perceptible representation may include a visual indication, an audible indication, or the like. The human-interface device 55525 may include a computer display. For example, the human-interface device 55525 may include a visual representation including text and/or images on a computer display. The human-interface device 55525 may include a text-to-speech device. For example, the human-interface device 55525 may include synthesized language prompt over an audio speaker. The human-interface device 55525 may communicate the computational data to the surgeon and/or surgical team. The human-interface device 55525 may include and/or be incorporated into any suitable device disclosed herein. For example, the human-interface device 55525 may include and/or be incorporated into a primary display 20023 of FIG. 2.

The notifications 55527 may include any device suitable for generating a perceptible indication that relevant computational data is available and/or has changed. The indication may include a visual indication, an audible indication, a haptic indication, and the like. The notifications 55527 may include non-verbal and/or non-textual indications to represent contextual data is available and/or has changed. For example, the alert system may include audio tones, visual color changes, lights, and the like. For example, the notification may include a haptic tap on a wearable device, such as a smartwatch worn by the surgeon. Notifications 55527 may include computational data, pre-surgical data, surgical data, and/or post-surgical data. Notifications 55527 may include a request from a machine learning algorithm for a HCP 55526 to provide feedback regarding data, a recommendation, an accuracy of an artificial intelligence model, an accuracy of training data, an accuracy of machine learning, a diagnosis, an indication of a problem, data generated by machine learning, a patient analysis, a conclusion regarding a patient analysis, a modification to a surgical device control program, a surgical device control program, wearable control program, any combination thereof, and the like. For example, notifications 55527 may request that HCP 55526 provide feedback regarding a surgical device control program that may be sent to surgical device 55522.

The surgical device 55522 may include any equipment employed for a surgical procedure (such as surgical systems 55511) that may have a configurable aspect to its operation. The configurable aspect of the equipment may include any adjustment or setting that may influence the operation of the equipment. For example, surgical device 55522 may have software and/or firmware adjustable settings. Surgical device 55522 may be hardware and/or structurally adjustable settings. In an example, the surgical device 55522 may report its present settings information to the computing system 55500. In an example, the surgical device 55522 may include an artificial intelligence model that may be deployed by computing system 55500, trained at computing system 55500, modified by computing system 55500, any combination thereof, and the like.

Example device settings for surgical device 55522 may include placement, imaging technology, resolution, brightness, contrast, gamma, frequency range (e.g., visual, near-infrared), filtering (e.g., noise reduction, sharpening, high-dynamic-range), and the like for imaging devices; placement, tissue precompression time, tissue precompression force, tissue compression time, tissue compression force, anvil advancement speed, staple cartridge type (which may include number of staples, staple size, staple shape, etc.), and the like for surgical stapling devices; and placement, technology type (such as energy devices, electrosurgery/laser surgery, mono-polar, bi-polar, and/or combinations of technologies), form-factor (e.g., blade, shears, open, endoscopic, etc.) coaptation pressure, blade amplitude, blade sharpness, blade type and/or shape, shears size, tip shape, shears knife orientation, shears pressure profile, timing profile, audio prompts, and the like for energy devices, for example.

Computational data collection 55530 may include data from wearable device 55524. Wearable device 55524 may include any configuration of hardware and software devices suitable for sensing and presenting patent parameters and/or biomarkers that may be relevant before, during, or after a surgical procedure. Such systems may be used by the patient for any amount of time prior to surgery, inside and outside of a medical facility. To illustrate, via an uncontrolled patient monitoring system, the patient may use a wearable heart-related sensor at during a surgical procedure. And/or, via a controlled patient monitoring system, a healthcare provider may monitor the same and/or analogous biomarkers using facility equipment during time of the surgical procedure. For example, the wearable device 55524 may provide data suitable for use in a contextual determination during and/or after surgery. Wearable device 55524 may include any wearable sensing system 20011 of FIG. 1 as described herein.

Computational data collection 55530 may include a wearable control program that may have been sent by wearable control programs 55520 wearable device 55524. Computational data collection 55530 may include an artificial intelligence model that may be sent to wearable device 55524.

The machine learning module 55514 may perform data preparation as described herein with the pre-surgical data collection 55528 (e.g., a dataset). In an example, the data preparation may further include the machine learning module 55514 receiving input from an HCP 55507 labeling a subset of the data records in the dataset for training a pre-surgical patient analysis model (e.g. a training dataset). The pre-surgical patient analysis model may be stored at and/or or included within Al model 55517. The pre-surgical patient analysis model may be a training data set with supervised machine learning for patient analysis 55518 (e.g., a probability of surgical complications during a surgical procedure). For example, machine learning 55514 may receive data to from pre-surgical data collection 55528 that may be used to train an ML model that may be stored at Al model 55517 and may be deployed at patient analysis 55518.

Those of skill in the art will recognize any suitable machine learning algorithm may be used to build the model 55517. For example, the input from HCP 55507 may include a "high risk" label when a patient's data record from patent records 55504 indicates a risk of surgical complications related to adhesions due to a history of multiple prior colorectal surgical procedures and pre-surgical biomarker measurement data from a pre-surgical sensing system 55502 or a wearable device 55506 indicating a probability of presence of chronic inflammation response. For example, the input from HCP 55507 may include a "medium risk" label when a patient's data record indicates a risk of surgical complications related to adhesions due to a history of multiple prior colorectal surgical procedures without any indication from pre-surgical biomarker measurement data that there is a probability of presence of chronic inflammation response. For example, the input from HCP 55507 may include label "low risk" a when a patient's data record indicates a risk of surgical complications related to adhesions due to a history of a single prior colorectal surgical procedure without any another indication of a probability of adhesion. The labels provided by HCP 55507 may be machine learning 55514 to train one or more models that may be used for patient analysis, modification and/or creation of surgical device control programs, and modification and/or creation of wearable control programs. The model may be stored at Al models 55517 and may be deployed at machine learning 55514, patient analysis 55518, surgical device control programs 55519, and/or warble control programs 55520.

Further, the input from HCP 55507 may include a notification level setting associated with each high-risk label, medium-risk label, or low-risk label. For example, a notification level setting may used by machine learning module 55514 to train an ML model to send a notification to HCP 55507 and/or HCP 55526 when the model may predict a high risk of surgical complication. In an example, a notification level may be used by the model when deployed at machine learning 55514 to send a notification to HCP 55507 when the model predicts a high risk of surgical complication. The HCP 55507 may respond to the notification with feedback, and the model may further be trained using the feedback. In an example, a notification level may be used by the model when deployed at patient analysis 55518 to send a notification to HCP 55526 and/or notifications 55527 when the model predicts a high risk of surgical complication. The HCP 55526 may respond to the notification with feedback, and the model may further be trained using the feedback.

The data preparation may also include the machine learning module 55514 receiving input from an HCP 55507 labeling a second subset of the data records in the dataset for validating an ML model (e.g., a validation dataset) with supervised machine learning.

Machine learning module 55514 may perform model training for the model. Machine learning module 55514 may perform model validation with the validation dataset after model may be deem trained (e.g., when a neutral network-based model's cost function has reached a global minimum).

Upon completing model validation, the machine learning module 55514 may perform model testing using a third subset of the data records in the dataset (e.g., an unlabeled dataset) for testing an ML model. The machine learning module 55514 may send predictions produced by the model to HCP 55507 for verification and/or HCP 55526. For example, the model may predict a high risk of surgical complication and an associated notification level of high-risk surgical complications. The machine learning module 55514 may send the high-risk prediction, notification level of high risk only, and decision points that may have led to such prediction (e.g., from the model 55517 trained with a decision tree machine learning algorithm).

In an example, based on the training dataset labeled by the HCP 55507, the model that may have been trained with a decision tree machine learning algorithm may learn a pattern (e.g., among other patterns) that a high-risk level of surgical complications may correlate with the combination of three or more prior colorectal surgical procedures and pre-surgical measurement data for at least one biomarker associated with a probability of chronic inflammation response (e.g., a high skin conductance level, a low tissue oxygenation level, and the like). Such a pattern may be a decision point in a decision tree algorithm-based model. The machine learning module 55514 may send a decision point along with the high-risk prediction and the notification level of high risk to the HCP 55507 and/or HCP 55526. machine learning module 55514 may send a decision point along with the high-risk prediction and the notification level of high risk to notifications 55527. In response, the HCP 55507 and/or HCP 55526 may provide a response verifying that the prediction is accurate. The verification may contribute to a success metric for meeting an accuracy parameter for deploying the model in a production environment (e.g. may be used on patients without supervision). A response from the HCP 55507 and/or HCP 55526 indicating the predication may be inaccurate may contribute to a failure metric for preventing model deployment due to inaccurate model predictions (e.g. may be used on patients with supervision).

The machine learning module 55514 may output the decision tree from the model. For example, the decision tree may be stored in Al models 55517. The decision tree may be sent to HCP 55507 and/or HCP 55526 to allow the decision tree to be verified holistically as opposed to one predication at a time.

Upon successful model testing with the test dataset, the computing system 55500 may deploy the model to a production environment production. For example, the model may be deployed to machine learning 55514, machine learning 55515, machine learning 55521, and patient analysis 55518. The deployed model 55517 may be further improved (e.g., for patient analysis purposes) in production. For example, patient analysis 55518, machine learning 55514, and/or machine learning 55515 may use feedback from a HCP 55508 to improve the model.

For example, the model may produce false negative predictions and/or false positive predictions. Feedback for such false negative and/or false positive predictions may be sent to the machine learning module 55521. In an example, the model may incorrectly predict a high risk of surgical complication. When the machine learning module 55521 sends an associated notification 55527, which may be sent to HCP 55526, the HCP 55526 may provide a response to the machine learning module 55521 indicating the prediction is a false positive. In such case, the machine learning module 55521 may not update the model threshold for predicting a positive prediction for surgical complications. The model may be stored and/or updated in Al models 55517 such that another deployment of the model may benefit from the feedback improvements.

In an example, the model may incorrectly predict no risk of a surgical complications (e.g., conflict data). The machine learning module 55521 may fail to send a notification, such as notifications 55527, to HCP 55526. HCP 55526 may not be provided with an opportunity to provide feedback. In such a case, the machine learning module 55521 may detect the error by checking the model prediction against the surgical outcome data from surgical system 55511 (e.g., which may be a surgical hub). The machine learning module 55521 may lower the model threshold for predicting a positive prediction for surgical complications to reduce the possibility of predicting false negatives. The model may be stored and/or updated in Al models 55517 such that another deployment of the model may benefit from the feedback improvement and/or detection of the error.

The machine learning module 55515 may perform data preparation as described herein with the surgical data collection 55529 (e.g., a dataset) for creating and training an ML model, which may be a surgical device control program model. The model may be stored and/or deployed at Al models 55517. The model may be deployed at machine learning 55515, machine learning 55521, and/or surgical device control programs 55519. In an example, the data preparation may further include creating a data field and appending it to a (e.g., each) data record in the dataset. The data field may indicate whether there was a surgical complication during a respective surgical procedure derived from surgical data from collected from a surgical system 55511 (e.g., a surgical hub).

The data field may indicate whether an operation of a surgical device or a wearable device may be improved (e.g. the device may have sub-optimally operated). The data field may serve as a desired output label for training the model with supervised machine learning for improving an ML model and/or a surgical device control program that may be determined and/or deployed at device control program 55519 to improve a surgical outcome. For example, the model may be deployed at surgical device control program 55519 and may be used to improve a surgical device program associated with surgical device 55522.

The machine learning module 55515 may perform model training, model validation, model testing for an artificial intelligence algorithm that may be used to create an ML model, such as a decision tree algorithm model. Those of skill in the art will recognize any other suitable machine learning algorithm may be used to build the model. The model may learn a pattern (e.g., among other patterns) that a surgical complication (e.g., a bleeding complication) occurs when a first condition and a second condition occur. The first condition may be that data from surgical sensing system 55509 and/or wearable device 55510 indicates at least one of: heart rate elevated above a threshold A, blood pressure above a threshold B, blood pH below a threshold C, or an edema measurement above a threshold D. The second condition may be that a control program associated with surgical device 55522 (e.g., a linear stapler) may be configured to compress tissue with a compression force below a threshold E. The model may learn another pattern (e.g., among other patterns) that a surgical complication (e.g., a bleeding complication) does not occur when the first condition and the second condition occur. A third condition may be that a control program associated with surgical device 55522 (e.g., a linear stapler) may be configured to compress tissue with a compression force above the threshold E.

Upon model testing using a test dataset, the computing system 55500 may deploy model to a production environment as a part of the machine learning module 55521. For example, the model may be deployed at machine learning 55514, machine learning 55515, machine learning 55521, Al models 55517, patient analysis 55518, surgical device control programs 55519, and/or wearable control programs 55520. During an operation in production, the model may detect a data pattern that the model may have learned during model training. For example, the model may receive input data indicating heart rate elevated above threshold A and indicating that a control program for surgical device 55522 is configured to apply a compression force below threshold E. In response, the model may predict a surgical complication and the machine learning module 55521 may update a deployed model, may update an ML model for generating a surgical device control program, may send updated parameters to the surgical device, or may send an updated surgical device control program to the surgical device to, for example, increase the compression force to be above threshold E.

The machine learning module 55515 may perform data preparation as described herein for creating and training an ML model, which may be an ML model for a wearable device such as wearable device 55510, using the pre-surgical data collection 55528 (e.g., a pre-surgical dataset) and the surgical data collection 55529 (e.g., a surgical dataset). In an example, the data preparation may further include creating a data field and appending it to a (e.g., each) data record in the dataset. The data field may indicate whether there may have been a surgical bleeding complication during a respective surgical procedure derived from surgical data from collected from a surgical system 55511 (e.g., a surgical hub). The data field may serve as a desired output label for training the model with supervised machine learning for adjusting a wearable control program, which may be stored at wearable control program 55520 and may be deployed at wearable device 55524, for improved sensed data relevancy.

For example, the machine learning module 55515 may perform model training, model validation, model testing for an ML model, such as a decision tree algorithm model. Those of skill in the art will recognize any other suitable machine learning algorithm may be used to build the model. The model may learn a pattern (e.g., among other patterns) that a surgical bleeding complication occurs (e.g., at a dissection/mobilization procedure step) when at least two conditions occur. One condition may be that pre-surgical data from pre-surgical sensing system 55502 and/or wearable device 55506 indicates at least one of: heart rate elevated above a threshold A, blood pressure above a threshold B, blood pH below a threshold C, or an edema measurement above a threshold D. Another condition may be that surgical data from surgical sensing system 55509 and/or wearable device 55510 indicates at least one of: heart rate elevated above a higher threshold A' (e.g., as compared to threshold A), blood pressure above a higher threshold B' (e.g., as compared to threshold B), blood pH below a lower threshold C' (e.g., as compared to threshold C), or an edema measurement above a higher threshold D' (e.g., as compared to threshold D).

The machine learning module 55515 may be configured to send update an ML model for a wearable control program. For example, machine learning module 55515 may update an ML model that may be deployed at machine learning 55514, machine learning 55515, machine learning 55521, wearable control programs 55520, wearable device 55506, wearable device 55510, wearable device 55524 and the like. The machine learning module 55515 may be configured to update a wearable control program that may be stored and/or deployed at wearable control programs 55520, wearable device 55506, wearable device 55510, and/or wearable device 55524. For example, an update may be sent to update the wearable control program of wearable device 55524 (e.g., configured for measuring heart rate, blood pressure, blood pH, and/or edema) when a surgical procedure (e.g., a sleeve gastrectomy procedure) is detected to have entered a dissection/mobilization procedure step. The update to the wearable control program may be to increase the data sampling rate (e.g., from once per minute to once per second). During the model operation (e.g., after model deployment) as a part of the machine learning module 55521 in production, such increased data sampling rate (e.g., during the dissection/mobilization) of biomarker measurement data related to bleeding complications may be sent to the HCP 55508 and/or HCP 55526 (e.g., via device 55525) to equip the HCP 55526 with more relevant data to prevent/mitigate potential bleeding complications.

Patient analysis 55518 may include software that may be used to provide analysis on a patient. For example, the analysis may indicate a probability of a surgical complication, a probability of surgical success, a diagnosis of a disease, a probability of a disease, a probability of a patient recovery, and the like. Patient analysis 55518 may include an ML model. The model may be stored outpatient analysis 55518 and/or deployed at patient analysis 55518. Patient analysis 55518 may include a number of models. For example, patient analysis 55518 may include one model for high blood pressure, a second model for normal blood pressure, and the third model for patients with diabetes. An ML model deployed at patient analysis 55518 may be from machine learning 55514, machine learning 55515, machine learning 55521, and/or Al models 55517. Patient analysis 55518 may include computational data.

Surgical device control programs 55519 may include software that may be used to provide control programs for surgical devices. Surgical device control programs 55519 may include device control programs, such as firmware, that may be stored for surgical devices. Surgical device control programs 55519 may include one or more parameters that may be used to configure, modify, operate, or control a surgical device. Surgical device control programs 55519 may include an ML model. For example, surgical device control programs 55519 may store an ML model that may be used for a surgical device, may deploy an ML model that may be used for a surgical device, or may update an ML model that may be used for a surgical device. An ML model deployed at surgical device control programs 55519 may be from machine learning 55514, machine learning 55515, machine learning 55521, and/or Al models 55517. Surgical device control programs 55519 may include computational data.

Wearable control programs 55520 may include software that may be used to provide control programs for wearable devices. Wearable control programs 55520 may include device control programs, such as firmware, that may be stored for wearable devices. Wearable control programs 55520 may include one or more parameters that may be used to configure, modify, operate, or control a wearable device. Wearable control programs 55520 may include an ML model. For example, wearable control programs 55520 may store an ML model that may be used for a wearable device, may deploy an ML model that may be used for a wearable device, or may update an ML model that may be used for a wearable device. An ML model deployed at wearable control programs 55520 may be from machine learning 55514, machine learning 55515, machine learning 55521, and/or Al models 55517. Wearable control programs 55520 may include computational data.

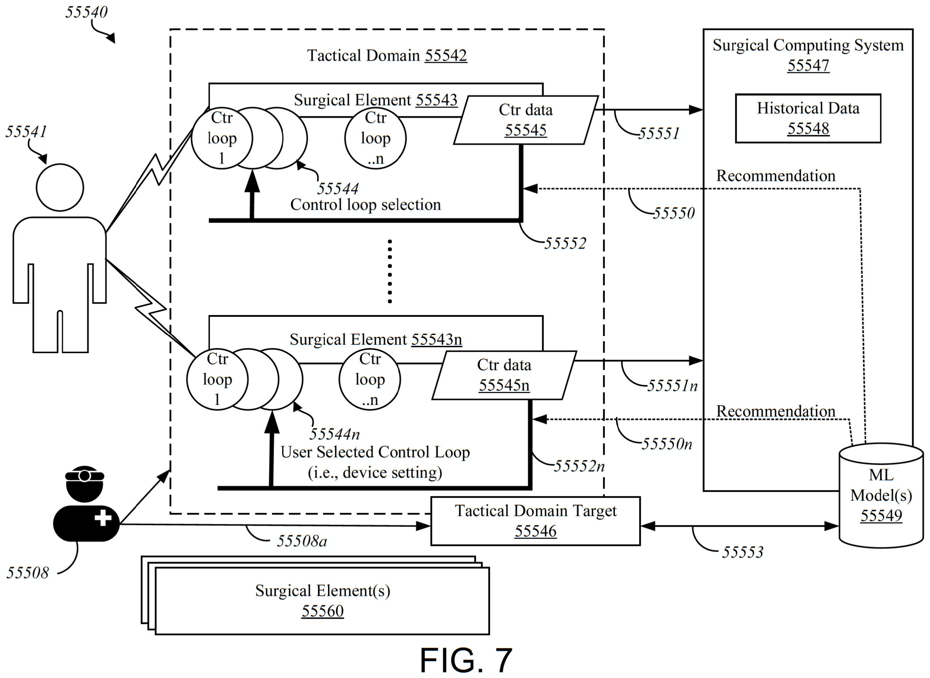

FIG. 7 is an example operational environment 55540 in which a surgical computing system 55547 may receive, among other things, control data 55545 (e.g., via 55551) and/or a tactical domain target 55546 (e.g., via 55553) to determine an optimized control loop 55544 for a surgical element 55543.

The operational environment 55540 may include a tactical domain 55542, a surgical computing system 55547, an HCP 55508, and/or a patient 55541 (although HCP 55508 is depicted in FIG. 7, HCP 55507, and/or 55526 of FIG. 6 may be interchangeably used). An operational environment 55540 (e.g., a tactical domain 55542) may infer one or more relationships between surgical elements 55543, 55543n, HCP 55508 a patient 55541, a tactical domain target 55546, and/or the like. The one or more relationships may be inferred based on for example, control data 55545, 55545n, historical data 55548, recommendation 55550, 55550n, and/or the like to create a tactical domain 55542 (also referred to hereinafter as tactical domain data). In examples, the operational environment 55540 may exclude one or more surgical elements 55560 from a tactical domain 55542 to optimize a procedure and/or efficiently process data.

A tactical domain 55542 may include tools, capabilities, data, and/or resources (e.g., HCP 55508, patient 55541) associated with a procedure. A tactical domain 55542 may include one or more surgical elements 55543, 55543n, configured to communicate with one another and/or with one or more components of an operational environment 55540 and/or a tactical domain target 55546 indicating a setpoint for the tactical domain 55542. Surgical elements 55543, 55543n, may include control loops 55544, 55544n, control data 55545, 55545n, and/or a selector 55552, 55552n.

A tactical domain 55542 may be generated and/or determined based on a ML model 55549 (e.g., of a surgical computing system 55547). A tactical domain 55542 may transmit control data 55545, 55545n (e.g., via 55551, 55551n) and/or a tactical domain target 55546 (e.g., via 55553) to a surgical computing system 55547. A tactical domain 55542 may receive a recommendation 55550, 55550n, associated with a portion of a procedure. The recommendation may be based on control data 55545, 55545n, a tactical domain target 55546, and/or the like. As an example, in response to a received recommendation 55550, a tactical domain 55542 may automatically adjust a control loop 55544 (e.g., via selector 55552) associated with a surgical element 55543, to optimize the performance of the surgical elements 55543, 55543n, and/or to efficiently perform a portion of a procedure (e.g., such that an HCP 55508 does not need to manually adjust the surgical element 55543).

A tactical domain 55542 may be determined and/or generated (e.g., by the surgical computing system 55547) based on one or more relationships associated with surgical elements 55543, 55543n (e.g., control loops 55544, 55544n, and/or control data 55545, 55545n), a surgical computing system 55547 (e.g., historical data 55548 and/or ML models 55549), an HCP 55508, a patient 55541, a tactical domain target 55546, and/or any other component of a operational environment 55540.

One or more relationships may be pre-defined (e.g., via a look-up-table as part of historical data 55548). In examples, a look-up-table may indicate a relationship between a primary surgical element 55543, a secondary surgical element 55543n, and/or a tactical domain target 55546 for a selected procedure. One or more relationships may be determined, for example, in response to a user input (e.g., a user input 55508a by an HCP 55508 via a graphical user interface (GUI), during a procedure and/or selected by an HCP 55508 and stored in historical data 55548 prior to a procedure). As an illustrative example, an HCP 55508 may indicate, in a look-up-table, that a core body temperature of a patient 55541 (e.g., a tactical domain target 55546) may be related to both a ventilator's change of a tidal volume (e.g., a primary surgical element 55543) and/or a heating blanket's power output (e.g., a secondary surgical element 55543a).

In examples, one or more relationships may be determined based on data (e.g., based on one or more components of a operational environment 55540 such as control data 55545, historical data 55548, a tactical domain target 55546, and/or the like). As an illustrative example, a surgical computing system 55547 may determine a relationship (e.g., based on an analysis of historical data 55548) between a core body temperature (e.g., a tactical domain target 55546), a ventilator (e.g., a primary surgical element 55543), and/or a heating blanket (e.g., a secondary surgical element 55543n). The surgical computing system 55547 may determine a relationship for example, based on data indicating that if ventilator increases the tidal volume of air to a patient, a heating blanket's power output may be increased to maintain the patient's core body temperature.

The tactical domain 55542 may receive a user input 55508a from an HCP 55508 (e.g., via 55508a). In examples, a user input 55508a may include an adjustment of a surgical element 55543 setpoint, a selection of a control loops 55544 associated with a surgical element 55543, a selection of a tactical domain target 55546, an indication of one or more steps associated with a procedure, the identification of one or more relationships (e.g., as described herein), and/or the selection, removal, and/or replacement of a surgical element 55543 (e.g., during a procedure). A user input 55508a may be received via a GUI, one or more control inputs (e.g., a knob, a button, and/or the like), and/or a connected device (e.g., a smartphone, wearable electronic device, a computer, and/or the like). In response to a received user input (e.g., an adjustment of a surgical element 55543), a surgical element 55543 may generate control data 55545 and/or a tactical domain target 55546. An adjustment to a surgical element 55543 may cause the control data 55545 and/or a tactical domain target 55546 to be transmitted (e.g., via 55551, 55551n, and/or 55553 respectively) to a surgical computing system 55547.

A surgical element 55543 may include one or more control loops 55544, control data 55545, and/or a selector 55552. Surgical elements 55543 may include (not illustrated in FIG. 7) sensors, instrumentation, and/or tools as described with reference to 20282 of FIG. 5. A surgical element 55543 may be used by an HCP 55508 and/or may operate autonomously to execute one or more actions associated with a procedure. As described herein, a surgical element 55543 may include, for example, robotic surgical systems, navigation systems, smart imaging systems, endoscopic and/or laparoscopic systems, energy scalpels, anesthesia machines, patient monitoring systems (pulse oximeters, blood pressure monitors, EKG monitors, EEG monitors, and/or the like), energy devices (e.g., electrosurgical units, laser surgery systems, and/or the like), infusion pumps, and/or the like. In examples, surgical elements 55543 may be wirelessly connected and/or physically connected (e.g., wired) to a network, a surgical computing system 55547, and/or one or more additional surgical elements 55543n. A surgical element 55543 may send control data 55545 to another computing system, to other surgical elements 55543n, and/or to a surgical computing system 55547 (e.g., via 55551) as described herein.

A tactical domain 55542 may include one or more control loops 55544 associated with a surgical element 55543. A control loop 55544 may change response characteristics of a surgical element 55543 (e.g., a behavior and/or an output characteristic of the surgical element 555343) based on an input from a patient 55541, an HCP 55508, a tactical domain target 55546, a recommendation 55550, 55550n, a network, other surgical elements 55543n, and/or a surgical computing system 55547. A control loop 55544 (e.g., an optimized control loop) may enable the surgical element 55543, an HCP 55508, and/or a patient 55541 to adapt, provide stability and control, improve precision and accuracy, improve consistency, and/or increase efficiency of an output characteristic of the surgical element 55543 during one or more portions of a procedure. As an illustrative example, an optimized control loop 55544 may monitor the force applied to a robotic instrument, and adjust the amount of force applied to prevent excess pressure that may damage tissue (e.g., a force-sensing instrument to measure a force applied during the membrane peeling process of a vitrectomy and membrane peeling).

Throughout a procedure, a tactical domain 55542 (e.g., via recommendation 55550, 55550n) may change and/or adjust an optimized control loop 55544 based on a recommendation 55550, 55550n (e.g., a one or more recommendations 55550 indicating one or more optimized control loops 55544 may be received during a procedure). For example, a recommendation 55550 may indicate a first control loop 55544 for a first portion of a procedure, and a second control loop 55544n for a second portion of a procedure. An optimized control loop 55544 may change, for example, based on a characteristics of a procedure, the patient 55541, the HCP 55508, the performance of a surgical element 55543, and/or the like (e.g., the length of a procedure, the number of complications associated with a procedure, the vulnerability of the patient, a specific hospital, a specific operating room, and/or the like). In examples, an optimized control loop 55544 may be different (e.g., response characteristics of the optimized control loop 55544 may be slower and/or faster) for a patient that is susceptible to hypothermia (e.g., an elderly patient, a pediatric patient, and/or the like) versus a patient that may be more resistant to hypothermia (e.g., a patient with a high body-mass index, patients with high basal metabolic rates, and/or the like).

A tactical domain 55542 may include control data 55545 associated with a surgical element 55543. Control data 55545 may include operational data and/or patient data (e.g., including pre-surgical data collection 55528, surgical data collection 55529, and/or computation data collection 55530 of FIG. 6). Operational data may include information associated with the surgical element 55543 during a procedure (e.g., an output characteristic of the surgical element 55543). For example, operational data may include the speed of a cutting tool, a selected control loop 55544 for a surgical element 55543, a voltage and/or current of an electrosurgical tool, a flow rate for an infusion pump and/or the like. Patient data may include real-time data associated with one or more physiological parameters of a patient during a procedure (e.g., a patient's heart rate, SpO2, temperature, and/or as described herein). Additionally and/or alternatively, control data 55545 may include historical data 55548 (not illustrated as part of surgical element 55543, 55543n), that may be associated with past operational data generated during the same and/or a similar procedure and/or patient data generated during the same and/or a similar procedure (e.g., a specific patient's data for one or more procedures and/or multiple patient's data for one or more procedures).

A tactical domain 55542 may include a tactical domain target 55546. A tactical domain target 55546 may be a setpoint and/or any other measured variable associated with the operational environment 55540 (e.g., associated with a surgical element 55543, a patient 55541, an HCP 55508, and/or the like). In examples, a tactical domain target 55546 may include one or more physiological parameters of a patient 55541 such as a core body temperature, a localized body temperature, oxygen saturation, blood pressure, respiratory rate, blood sugar, heart rate, hydration state, and/or the like. In examples, a tactical domain target 55546 may include a setpoint and/or one or more measured variables associated with a surgical element 55543 such as a voltage and/or current for an electrosurgical tools, flow rate for an infusion pump, power outputs for a laser device, concentration amount for an anesthesia machine, tidal volume for a ventilator, and/or the like. A tactical domain 55542 may receive a tactical domain target 55546 from an HCP 55508 (e.g., via 55508a) and/or from a surgical computing system 55547 (e.g., via 55553). A tactical domain 55542 may determine a tactical domain target 55546 based on one or more surgical elements 55543, 55543n. As an example, an HCP 55508 may select, as a tactical domain target 55546, a temperature setpoint to control the core body temperature of a patient during a procedure.

An operational environment 55540 may include a surgical computing system 55547. A surgical computing system 55547 may include historical data 55548, and/or ML models 55549. A surgical computing system 55547 may receive control data 55545, 55545n (e.g., via 55551, 55551n) from a surgical element 55543, 55543n, and/or a tactical domain target 55546 from a tactical domain 55542 (e.g., via 55553). A surgical computing system 55547 may determine, based on the received control data 55545, 55545n, historical data 55548, and/or a tactical domain target 55546, a recommendation 55550, 55550n for a surgical element 55543 and/or 55543n. A recommendation 55550 may be transmitted wirelessly and/or via a wired connection. A recommendation 55550 may be determined based on one or more ML models 55549 and/or based on one or more relationships as described herein. A recommendation 55550 may include for example, an indication of adjustment and/or a selection of an optimized control loop 55544 associated with a portion of a procedure, an indication of a surgical element 55543 to be used during a portion of a procedure, an indication of a surgical element 55560 to be excluded from a tactical domain 55542, and/or an indication of a conflict associated with one or more surgical elements 55543, 55543n, during a portion of a procedure.

A surgical computing system 55547 may include historical data 55548. Historical data 55548 may include information associated with past recommendations, historical control data from one or more surgical elements 55543, 55543n, data associated with an HCP 55508 (e.g., procedural time, specific surgical elements used by an HCP 55508 during a procedure, and/or the like), historical data 55548 associated with a patient's physiological parameters (e.g., biomarkers), multiple patients physiological parameters associated with a procedure, and/or the like (e.g., including pre-surgical data collection 55528, surgical data collection 55529, and/or computation data collection 55530 of FIG. 6).

A surgical computing system 55547 may include ML models 55549. An ML model 55549 may be trained to achieve a tactical domain target 55546 by generating a recommendation 55550 (e.g., including a selection of an optimized control loop 55544) as described herein. ML model 55549 may generate individual training data items that form training data. Training data items may correspond to a particular portion of a procedure, include historical data 55548, and/or the like. An ML model 55549 may train an ML model to generate and/or determine an output based on control data 55545, based on a portion of a procedure, based on a tactical domain target 55546, and/or the like.

In examples, an ML model 55549 may be trained to predict an optimized control loop 55544, 55544n for one or more surgical elements 55543, 55543n. The optimized control loop 55544 may be determined, such that a tactical domain 55542 achieves a tactical domain target 55546 in an efficient, safe, controlled, and/or economical manner (e.g., during a procedure). The determined optimized control loop 55544 may be sent to a tactical domain 55542 via recommendation 55550, where the tactical domain 55542 (e.g., the surgical element 55543) may automatically adjust a control loop 55544 via selector 55552.

In examples, an ML model 55549 may be trained to determine an optimized surgical element 55543. The optimal surgical element 55543 may be determined for a tactical domain 55542 and/or for use during a portion of a procedure. The recommendation 55550 may be sent to the tactical domain 55542, where the tactical domain may automatically include and/or remove one or more surgical elements 55543, 55543n, and/or 55560 from the tactical domain 55542. In examples, the recommendation 55550 may be sent to an HCP 55508 (e.g., via a GUI), where the HCP 55508 may determine whether to include and/or remove a surgical element 55543 based on the recommendation 55550. As an illustrative example, an ML model 55549 may determine that a specific cutting tool (e.g., a specific serial number) is the most reliable tool from a list of similar cutting tools, that a type (make and/or model) of ventilator provides optimized control during a procedure, and/or the like, and send an indication of the reliable tool, via a GUI, to an HCP 55508.

In examples, an ML model 55549 may be trained to anticipate conflicts between surgical elements 55543, 55543n, and/or the like. An anticipated conflict may be determined for a tactical domain 55542 and/or for portion of a procedure. The recommendation 55550 may be sent to the tactical domain 55542, where the one or more surgical elements 55543, 55543n may resolve the conflict based on the recommendation 55550, 55550n. In examples, an ML model 55549 may provide a recommendation 55550, 55550n to avoid contact between one or more movable arms of a robotic system during a procedure. In examples, the recommendation 55550 may be sent to an HCP 55508 (e.g., via a GUI) where the HCP 55508 may determine how to resolve the conflict, and/or validate whether the conflict actually exists.

Identifying trends associated with ML algorithms and/or ML enhancement of data or displays may be described herein. In examples, the description herein may be used to generate historical data 55548, and/or may be used as training data for a ML models 55549.

Separation of data into known buckets to enable an Al/ML model may be provided. Al/ML identification of visualized shapes may be described herein. Real-time removal of tissues, instrument or organs based on ML identification of the boundaries of the shapes and/or re-insertion of a missing environment may be provided. A real-time projection or by adding a virtual image over real-time imaging based on an ML adaptation of the perspective may be provided. In examples, overlaying an image may provide an ability to separate data into known buckets to enable and support Al/ML - automatic annotation (e.g., a system may label images such that they may be queried by a user). Known buckets can include HCP labels, contextual real-time tagging, boundary flagging around activations, and/or multi-variable flagging.

In examples, an HCP 55508 (e.g., a surgeon) may label one or more images. The system 55547 may utilize surgeon labeled images as a method for bypassing system-driven annotation. HCP 55508 annotation may allow the system 55547 to take advantage of surgeons who prefer to manually annotate images, video, and/o the like.

Contextual real-time tagging may be provided, which may include procedure step integration and/or operating room equipment integration (e.g., surgical element 55543). Procedure step integration may include a camera or system capturing a data (e.g., a video or images). The data may be integrated in with the system 55547 (e.g., a procedural planning system), such that the data (e.g., an image or video) is tagged with metadata relating to the current step or other metrics in real-time as opposed to post-processed. Operating room equipment integration may include a system tagging and/or annotating the data (e.g., video and/or images) with data to show when energy activations occur, and other discrete events within the operating room.

Boundary flagging around activations may be provided herein. In examples, a decoupling of the analysis may allow the ML algorithms (e.g., ML models 55549) to be more specific or constrained. Decoupling may reduce the complexity of ML algorithms (e.g., ML models 55549) and execution time of them.

Multi-variable flagging may be described herein. In examples, the system 55547 may generate flags based on captured data (e.g., video or images). The system 555547 may compile the flags, to detect (e.g., at a later point in time) the contextual annotation. In examples, the system 55547 may detect how bright an image is, clarity of the image, amount of red in the image, instrument detection, and/or the like. The flags may contribute to understanding of the context long term.

Multi-variable flagging may include the identification of liver photons. As an illustrative example, protons versus photons for unresectable hepatocellular carcinoma may be provided. Liver decompensation and overall survival results may include a median follow-up of 14 months. Of 133 patients with a median age of 68 years and/or 75% male, 49 (37%) were treated with proton radiation therapy. Proton radiation therapy may be associated with improved results (adjusted hazard ratio, 0.47; P = .008; 95% confidence interval [Cl], 0.27-0.82). The median results for proton and photon patients was 31 and 14 months, respectively, and the 24-month results for proton and photon patients was 59.1% and 28.6%, respectively. Proton radiation therapy was also associated with a decreased risk of non-classic radiation-induced liver disease (odds ratio, 0.26; P = .03; 95% Cl, 0.08-0.86). Development of non-classic RILD at 3 months was associated with the worse results (adjusted hazard ratio, 3.83; P < .001; 95% Cl, 2.12-6.92). There may be no difference in locoregional recurrence, including local failure, between protons and photons.

In examples, a system 55547 may improve data flow and/or storage via network traffic analysis and/or control, adjusting storage rate and/or location, selective inter connectivity, and/or by being situationally dependent. In examples, a system 55547 may shift between real data collection and post-op collection of an autonomous systems to enable collection of more important things (i.e. a surgical element may transmit wirelessly real time or the surgical element may be asked at the end of the procedure to download its dataset).

In examples, quasi-synthetic data may include data that is generally accurate, and/or may execute a function to make the data accurate (e.g., completely right). In some examples, quasi-synthetic data may be generated when/if there is not enough data and/or sensor information present (e.g., a system 55547 may revert to an ML model 555549 for a next action during a procedure). In examples, one or more sensors may be available, and/or the system 55547 may use and ML model 55549 to inform the system and/or an HCP 55508 of a determination for an efficient and/or successful outcome. In some examples, a subjective choice may include synthetic data (e.g., data that is invented/created by something else).

A transfer algorithm adaptation outside real-time may be provided. Solving one or more equations that may be needed and/or introducing equations to the system may be provided herein. Developing an Al/ML model, and/or how one or more unknowns are solved (e.g., based on settings and/or shifting thresholds) may be provided.

For example, patterns in results that a user may select for tactical decisions (e.g., a tactical domain target 55546) for assistance in future decision making may be described herein. Collection of user choices when requested (e.g., required) for automated decision making with resulting outcomes, from multiple surgeries, to provide weighted suggestions for future decisions may be provided. Aggregation of user derived decisions from one or more data streams and the outcomes from patients to form suggestions for future user choices may be provided. The more data streams than closed loop control systems (tactical decisions below) which have more than one choice and were presented to the user for input may be provided. The user may be presented with information including a display of multiple data streams and how the multiple data streams interact (e.g., one or more relationships). The display may present the proposed improvements to the operational algorithms (e.g., a recommendation 55550, 55550n).

ML and/or trending aggregation may be provided herein to obtain strategic decisions. Long term adaptations of the control or display systems via large dataset ML aggregation or trending may be provided herein. The AI/ML model (e.g., ML model 55549) may be acting on a current procedure, and/or on one or more procedures that occur in the future. In examples, there may be instances where ML model 55549 would use data from a current procedure versus from a procedures of a specific type. For example, a sleeve standard of care may include an offset for staple lines of about 20 degrees, to follow greater curvature of stomach. After placing lines, the actual angle of offset may be 10-40 degrees. Surgeries where the actual angle is 25-35 may have the fewest complication instances. As a result, a feedback loop (after a threshold number of procedures) may recommend a 30 deg offset. The placement of staple lines at 30 degrees may be a strategic decision.

Non-real time aggregation pattern identification may be described herein. Adjustments to a procedure recommendation, based on ML datasets from patient data and/or surgeon data may inform a behavior that differs from the standard response (e.g., included as part of historical data 55548).

Data sets may be used to make a strategic decision. A strategic decision may be different than a tactical decision. In examples, comparison of real-time data resulting in a tactical decision over time may be used to make strategic decisions within a single surgery and/or between surgeries. In examples, a tactical decision may come from real time data during a procedure. For example, patient monitoring (a ventilator), the pressure inside tumor/chemo tip, a cone beam CT (e.g., a tumor volume filled), a chemo pump (e.g., drug volume delivered). In examples, the pressure rising within tumor may indicate that a drug delivery may be stopped to prevent spray out.

A system 55547 may compare real time data to a broader intra-surgery database (e.g., historical data 55548). A database may exist. Data may be generated based on a surgeon, one or more procedures (e.g., all procedures) from a piece of equipment, and/or as described herein. In examples, the pressure rising within tumor may indicate that an HCP 55508 may, instead of repositioning probe, ablate instead of delivering chemo (e.g., data from previous procedures informs spray out is bad, and a system may indicate a decision to ablate instead of deliver chemo). In examples, a tumor may have be less homogenous than initially estimated. A system 55547 may generate a new procedure plan based on new information from a patient 55541. In examples, a patient 55541 with a history of cancer may have a limit to an amount of chemo administered. Regardless of tumor saturation, a decision to stop chemo once a threshold volume is achieved may be indicated by the system 55547.

A system 55547 may utilize trends or patterns within and/or between data streams, procedures, patients 55541, and/or physicians to generate strategic decisions (e.g., recommendation 55550). In examples, raw contextual data (e.g., historical data 55548 as described herein) could be used to identify patterns within the data streams, to find coupled feeds that enable the prediction of one feed by the leading feed of another stream. Data may indicate that a current treatment approach is not the best approach (e.g., as a result an HCP 55508 may should change an approach). In examples, an HCP 55508 may have an approach angle and/or a tumor approach direction that may have an implication on a tumor fill. An approach angle may determine whether a procedure is endoscopic and/or trans-parenchymal. In examples, an HCP 55508 may change from irradiation to cryo-ablation due to an issue with chemo delivery (e.g., patient outcomes are the same but a is lower, patient outcomes are the same but a procedure is lower risk, and/or patient outcomes are the same but a surgeon effort is less). In examples, a needle depth and/or drug pressure may be related (e.g., the needle moves beyond 60% depth, a spray out may occur). In examples, a strategic decision (e.g., a recommendation 55550) may include inserting the needle depth to about 30% depth.

There may be a decision that is not based on previous sets of AI/ML data. Previous data set may include strategic implications on one or more steps, procedures, placements of surgical instruments, and/or the like, that may improve surgical outcomes. In examples, where trocars and/or equipment may be placed for efficient use may be determined by Al/ML models. In examples, previous procedure steps may define one or more constraints for a stomach tumor procedure. For example, a tumor may be removed from peritoneum, and/or the amount of a tumor dissected may define an effort requested to remove a segment. In examples, an angle of a sleeve gastrectomy line may be defined where the next staple line will be placed. The system may generate an indication to an HCP 55508, providing a range that the next staple line may be placed at. In examples, a system 55547 may determine, for a tissue tension on a staple line, the amount of mobilization performed to define acceptability of a circular line. In examples, for geometry and/or anatomy, a trocar placement may be based on one or more adhesions. For examples, a trocar placement may be based on a tumor size, a number of needles, one or more feedback loops to fine-tune practice guidelines (e.g., control loops 55544), and/or the like.

Continuous improvement of the ML algorithm based on new incoming data and verification of an Al/ML algorithm (e.g., ML model 55549) may be provided herein. In examples, an HCP 55508 may determine whether to use a first type of energy device or a second type of energy device to clamp and/or fire onto tissue. An algorithm may suggest an approach (e.g., a smaller versus a larger effect on tissue). Data from one or more data streams (e.g., surgical elements 55543) may identify whether the first type of energy device or the second type of energy device is better than an Al/ML preferred technique. In examples, an algorithm may learn based on accepted metrics (e.g., less bleeding, quicker recovery, and/or the like). In examples, a skill assessment of an HCP 55508 (e.g., a surgeon) may be generated (e.g., as part of historical data) and/or compared to provide procedure improvement suggestions.

In examples, a system may examine the trends of the previous procedures (e.g., historical data 55548), looking for patterns in the HCP (e.g., surgeon) techniques, selections of tools and access, anatomic landmark alignment, patient characteristics, patient biomarker reading (e.g., BMI, blood pressure, blood sugar, height, weight, stature, and/or the like) and the outcomes and complication from the previous surgeries, to present the surgeon with a viable choice in real-time and/or to provide context or potential implications of a choice to aid an HCP in their current choice.

A determination of a sleeve pouch size and/or an initiation location of a sleeve may be provided. In sleeve gastrectomy the size, shape, and/or other aspects of the pouch may relate to a patient's outcome, the difficulties of performing the procedure, and/or the probability of post operative complications. If the real-time data streams (e.g., via surgical elements 55543 and/or the like) provide multiple choices for the surgeon for a specific patient being scanned. Results and issues associated with previous surgeries (e.g., either similar patients and/or one or more patients for an HCP, for a surgical center, and/or for a geographic region) may be used to provide the HCP (e.g., the surgeon) with context, predictions, and/or suggestions as described herein. Real-time data streams may relate to the particular constraints of a patient, the approach, the skill level of the HCP, aspects of the general population, techniques, and/or instruments being used. In examples, one or more contributing factors (e.g., criteria that may be included in historical data 55548) may include, for a specific patient and/or for past patients, a stomach thickness, medications, a sleeve starting location from the pyloric sphincter, co-mobilities, a number of intersections and/or an angle size, a pouch size, a boogie size, and/or a uniformity of sleeve size (e.g., the sleeves may be uniform, and/or close to the esophageal sphincter as one moves parallel to the lesser curve, and/or the staple lines around the distance from the incisura-angularis may make the sleeve less than uniform in the distal portion of the sleeve, which may not be desirable), and/or buttress or not. In examples, one or more outcomes may include a durability or weight loss, post operating complications, a time, and/or an impact on patient recovery time.

AI/ML models (e.g., ML models 55549) may have different levels. In examples, ML may include large dataset with limited bounding. In examples an Al/ML model may determine how to parse data. In examples, Al/ML model observes a procedure, a behavior, and/or may preemptively make a change/decision. In examples, a surgical element 55543 may perform a behavior multiple (e.g., 18) times, the AI/ML model may indicate, to the system 55547 (e.g., indicate to the tactical domain 55542 via recommendation 55550) to set up differently based on the behavior. In examples, Al/ML models may include patterns. For an Al/ML model to generate an output (e.g., a recommendation 55550) in real time, verification and validation of an updated model must be achievable against a prior defined data set. In examples, an Al/ML model may be assessed based on new data and/or preexisting data. For example, as an HCP 55508 (e.g., a surgeon) makes decisions, the contextualized data may be incorporated into a dataset from the HCP 55508. This data may be used in training data (e.g., as described herein) to update an AI/ML model. An ML model 55549 may be verified against historical data 55548 which has been previously confirmed. As a result, this ensures that the ML model 55549 continues to meet the verification burden that the model was previously established with, but provides the model flexibility to continue to adapt.

Real time aggregation of HCP 55508 decisions (e.g., surgeon decisions) into a data set for model training and/or verification may be provided (e.g., as part of historical data 55548). In examples, as an HCP 55508 (e.g., a surgeon) makes decisions, contextualized data is incorporated into a dataset from the HCP 55508. This data may be assumed to have a valid output associated with it based on the HCP 55508 decisions. As a result, the data may be utilized in training, updating, or verifying an existing model based on a surgical outcome. As the quantity of surgical decisions becomes large enough, the model may begin to self-train and/or verify itself within its given bounds.

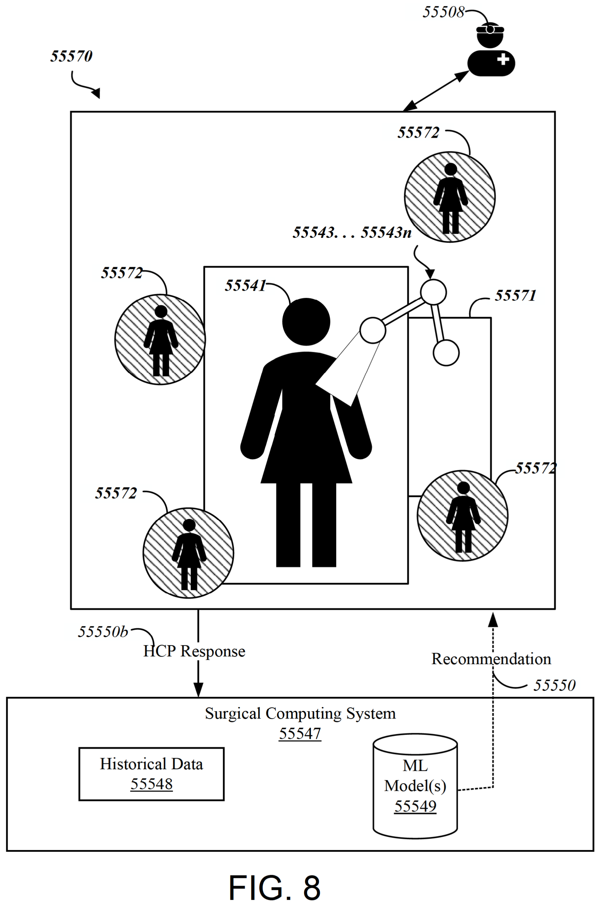

FIG. 8 illustrates an example user interface display 55570 indicating one or more conflicts as determined by a surgical computing system 55547 of FIG. 7. The display 55570 may be provided to an HCP 55508. The display 55570 may notify an HCP 55508 of potential a conflict and/or request that the HCP 55508 resolve a potential conflict associated with a procedure. In some examples, the display 55570 may be a heat map indicating one or more potential conflicts.

The example display 55570 may include a patient 55541, a robotic system 55571, and/or one or more potential conflicts zones 55572. The example display 55570 may be representative of an endoscopic procedure and/or any other procedure, where a robotic system 55571 may be equipped with one or more surgical elements 55543, 55543n (e.g., a camera, a suction tool, a robotic arm, and/or the like).

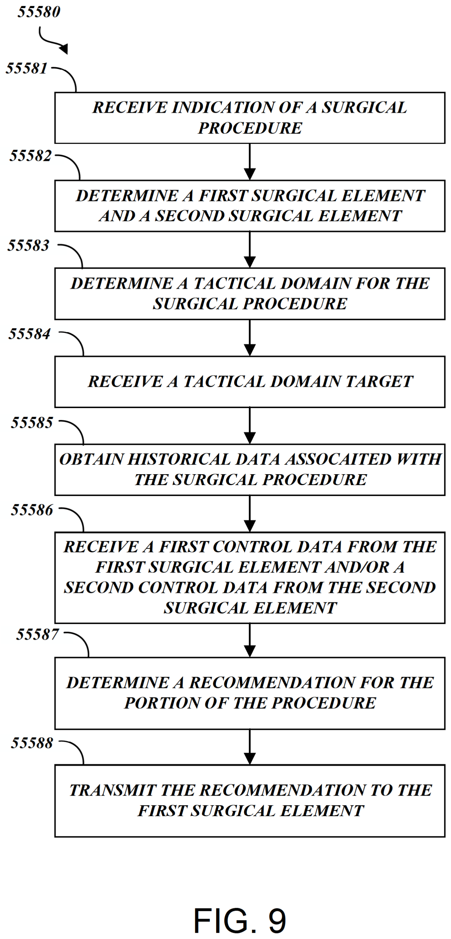

The display 55570 may include one or more conflict zones 55572. The conflict zones 55572 may be as part of a recommendation 55550 generated with reference to block 55587 of FIG. 9. Conflict zones 55572 may indicate a physical conflict area within an operating room, where an HCP 55508 may interfere with a robotic system 55571 during a portion of a procedure and/or where one or more surgical elements 55543, 55543n, may interfere with one another (e.g., if the HCP 55508 were to enter the conflict zone 55572). The conflict zones 55572 may be determined based on historical data, control data, and/or operational data as described herein. In examples, historical and/or control data may include the position, speed, and/or attitudes of surgical elements 55543, 55543n, and/or the movement of HCPs 55508, and/or a patient 55541 over time, during past procedures and/or during a current procedure.

The number and/or position of conflict zones 55572 may be modified based on one or more conditions of a patient 55541, an HCP 555508, surgical elements 55543, 55543n, and/or the like. For example, as a procedure progresses, an ML model 55549 may determine that a location may no longer be a conflict zone 55572 and remove the indication of a conflict zone 55572 from the display 55570. In examples, the display 55570 may receive a user input from an HCP 55508, confirming the validity of one or more conflict zones 55572, and/or acknowledging the existence of a conflict zone 55572. In response to a user input (e.g., via HCP response 55550b), the surgical computing system 55547 may further train an ML model 55549 based on the validity of one or more conflict zones 55572 for a procedure.

As described with reference to FIG. 8, an intra-procedure collision and/or position tracking may be utilized in an ML model (e.g., ML models 55549). In examples, an intra-procedure collision and/or position tracking method (e.g., as provided in display 55570) may define a context and data-focused path planning. As operating rooms become more connected and data centric, data may be collected regarding robotic system 55571 positions, speeds, device attitudes, movement of personnel, and/or knowledge of relevant locations, techniques, and outcomes for each step in the procedure. Data may be used to define a temporospatial heatmap of robotic positions (e.g., as illustrated in display 55570) to show common locations for the robot as they relate to a given procedure and approach.

Common and/or expected robotic locations (e.g., conflict zones 55572) at each given point in time along the course of a procedure may receive a positive numerical offset to define the given point as desirable or an usual position. Positions over time where the robot encounters sensitive and/or irrelevant organ structures, or is close to collision within or outside of the body (e.g., conflict zones 55572) may receive a negative numerical offset (e.g., to define an undesirable position). An outcome of the procedure and/or specific actions therein may be considered to add a multiplicative weighting value to appropriate positions in time (e.g., as part of ML models 55549). In examples, if an operating location on a gallstone removal results in a good outcome such as reduced bleeding (e.g., relative to expected) or good post procedure healing outcomes, the operating location may receive a positive multiplier to indicate that the position and time location were of unique advantage to procedural outcomes. If the operating location is associated with a negative outcome (e.g., like excessive bleeding), a fractional or negative multiplier may be added to reduce the desirability (e.g., fractional) of the operating location in time and/or make the operating location entirely undesirable (e.g., a negative outcome as depicted in conflict zones 55572).

An operating position in time and space (e.g., throughout a procedure) may include a numerical offset (e.g., +6, -2) that may define desirability of operating and/or moving the robotic system 55571 or end effector into that operating position based on frequency or knowledge of a dangerous or uncommon situation. A position may or may not receive a weighting value (a multiplier, i.e. x0.5 or x-1.0) that corresponds to patient health outcome of an action performed or related to that position in time. A position may be multiplied by the numerical offset in order to determine a gross overall desirability score for the heatmap (e.g., as provided in display 55570).

Due to the numerical nature of the heatmap (e.g., display 55570), multiple heatmaps may be overlaid via simple addition and/or overlay. Additional robotic systems 55571 in the operating room may increase the number of conflicting robot's desired positions (e.g., conflict zones 55572) and/or may provide a negative offset (e.g., as the likely presence of an additional robotic system 55571 in that space in time may result in possible conflicts and thus a lesser desirability assigned to that position). Multiple heatmaps may be summed to account for multiple robotic paths and/or obstacles or unique considerations within the operating room. The sum of heatmaps may be a net temporospatial heatmap for the robot in question (e.g., as provided in display 55570). Net heatmaps may be overlaid within the HCP's console (e.g., surgeon console) for the benefit of the HCP 55508 to provide additional information or to directly guide the motion of the robot or path plan.

In examples, a system (e.g., surgical computing system 55547) may annotate the number of actual conflicts compared to system predicted number. The system 55547 may predict an anticipated number of conflicts and/or detect a conflict in a procedure. The system 55547 may interrogate an HCP 55508 regarding the magnitude and occurrence of a conflict. The result may be saved in historical data 55548, including the actual and/or predicted or anticipated outcomes to adapt an AI/ML algorithm (ML models 55549) for an optimized prediction.

In examples, a system 55547 may provide a GUI (e.g., display 55570) to an HCP (e.g., a surgeon or other surgical staff) with a sequence of predicted conflicts (e.g., conflict zones 55572). The system 55547 may request that the HCP 55508 determine whether or not the one or more predicted conflicts 55572 occurred, input a number and/or type of actual conflicts, and/or input an adjustment to one or more conflicts to better inform the algorithm in future (e.g. distributed learning). The system 55547 may receive the HCP 55508 input via HCP response 55550b, including patient outcomes to improve predictions of conflicts. In examples, cameras in an operating room may observe and/or respond to a conflict instead of a user (e.g., an HCP 55508).

Adaptation of post-surgical historical data 55548 to reflect new anatomic configurations may be provided herein. In examples, historical data 55548 may include a post-surgical removal of tissues to fit the patient, procedure, outcome with the intervention integrated in the data. In examples pre-op information may be altered once an object is removed by the HCP 55508 (e.g., a surgeon). In examples, (e.g., the removal of gallstones) there may be an instance where an HCP (e.g., the surgeon) overlays a pre-op CT over a real time scope view of the patient. An HCP 55508 may remove the gall bladder via the connection to the common bile duct. Once the gall bladder is removed, the HCP 55508 may return to the bile duct and small intestine to remove remaining stones that were not extracted. If the HCP 55508 is interested in a CT overlay of the gall bladder, the gall bladder may be removed in the CT display so that the system 55547 displays relevant anatomy.

Smart system decisions on subjective interpretation using one or more linked smart data streams may be provided herein. Trends or patterns that may enable smart decision may improve stone identification thru external CT and/or ultrasound. A system may select a main threshold for a stone versus a stone in a gallstone identification. The system 55547 may handle indeterminate shapes and/or probabilities by an image including a dotted outline with the probabilities by identifying and coupling landmarks from multiple images and/or align or distort of the images based on the coupling.

FIG. 9 is a flow chart of an example optimized control loop selection routine 55580 illustratively implemented by a surgical computing system 55547 of FIG. 7. The routine 55580 begins at block 55581.

At block 55581, the surgical computing system 55547 may receive an indication of a surgical procedure. An indication of a surgical procedure may be received from an HCP 55508, a tactical domain 55542, and/or a surgical element (e.g., surgical element 55543 of FIG. 7). In examples an indication of a surgical procedure may be determined by the surgical computing system 55547. An indication of a surgical procedure may include, for example, a specific step, a sub-step, an operation, an act, and/or the like, for a surgical procedure, diagnostic procedure, therapeutic procedure, preventative procedure and/or the like. As a specific example, an indication of a surgical procedure may include administering anesthetics to sedate a patient during an endoscopic procedure and/or preparing an area of a patient's body for an incision.

At block 55582, the surgical computing system 55547 may determine a first surgical element and a second surgical element (e.g., surgical element 55543, 55543n of FIG. 7). The surgical computing system 55547 may determine the first surgical element and/or the second surgical element based on the received indication of a surgical procedure. The surgical computing system 55547 may determine the first and/or second surgical element based on an indication of one or more surgical elements from an HCP 55508, another network, and/or the like.

At block 55583, the surgical computing system 55547 may determine a tactical domain 55542 for the surgical procedure. As described herein, a tactical domain 55542 may include tools, capabilities, and/or resources to conduct a portion of a procedure. A tactical domain 55542 may include a network of surgical elements 55543, 55543n configured to communicate with each other and/or with one or more components of an operational environment 55540. For example, a tactical domain 55542 may include surgical elements 55543, 55543n, control loops 55544, 55544n, control data 55545, 55545n, a tactical domain target 55546, and/or a selector 55552, 55552n of the one or more control loops 55544, 55544n associated with a portion of a procedure. A tactical domain 55542 may be derived (e.g., generated) based on an ML model 55549 (e.g., of a surgical computing system 55547), and/or determined by an HCP 55508.

As described herein, a tactical domain 55542 may be determined and/or generated (e.g., by the surgical computing system 55547) based on one or more relationships associated with surgical elements 55543, 55543n (e.g., control loops 55544, 55544n, and/or control data 55545, 55545n), a surgical computing system 55547 (e.g., historical data 55548 and/or ML models 55549), an HCP 55508, a patient 55541, a tactical domain target 55546, and/or any other component of an operational environment 55540.

As described herein, one or more relationships may be pre-defined (e.g., via a look-up-table as part of historical data 55548). In examples, a look-up-table may indicate a relationship between a primary surgical element 55543, a secondary surgical element 55543n, and/or a tactical domain target 55546 for a selected procedure. One or more relationships may be determined, for example, in response to a user input (e.g., a user input 55508a by an HCP 55508 via a graphical user interface (GUI), during a procedure and/or selected by an HCP 55508 and stored in historical data 55548 prior to a procedure).

At block 55584, the surgical computing system 55547 may receive a tactical domain target 55546. A tactical domain target 55546 may be determined by a tactical domain 55542, an HCP 55508, and/or generated by the surgical computing system 55547. As described herein, a tactical domain target 55546 may be a setpoint and/or any other measured variable associated with one or more surgical elements 55543 and/or a patient 55541. In examples, a tactical domain target 55546 may include one or more physiological parameters of a patient 55541, such as a core body temperature, a localized body temperature, oxygen saturation, blood pressure, respiratory rate, blood sugar, heart rate, hydration state, and/or the like. In examples, a tactical domain target 55546 may include a setpoint and/or one or more measured variables associated with surgical elements 55543 such as a voltage and/or current for an electrosurgical tools, flow rate for an infusion pump, power outputs for a laser device, concentration amount for an anesthesia machine, tidal volume for a ventilator, and/or the like.

At block 55585, the surgical computing system 55547 may obtain historical data 55548 associated with the surgical procedure. The historical data 55548 may include past recommendations, historical control data from one or more surgical elements 55543, data associated with an HCP 55508 (e.g., procedural time, a surgical element 55543 used by an HCP 55508 during a procedure, and/or the like), historical data associated with a patient's physiological parameters (e.g., biomarkers) and/or multiple patients physiological parameters associated with a procedure, and/or the like.

At block 55586, the surgical computing system 55547 may receive a first control data (e.g., control data 55545) from the first surgical element and/or a second control data (e.g., control data 55545n) from the second surgical element. As described herein, first and/or second control data may include operational data, historical data, and/or patient data. Operational data may include information associated with a surgical element during a procedure. For example, operational data may include the speed of a cutting tool, a selected control loop for the surgical tool, a voltage and/or current of an electrosurgical tool, a flow rate for an infusion pump and/or the like. Historical data may include data associated with past operational data generated during similar procedures and/or patient data generated during a procedure (e.g., a specific patient's data for one or more procedures and/or multiple patient's data for one or more procedures). Patient data may include real-time data associated with the health of a patient during a procedure (e.g., one or more physiological parameters of a patient such as a heart rate, SpO2, temperature, and/or the like).

At block 55587, the surgical computing system 55547 may determine a recommendation 55550 for a portion of a procedure as described herein. The recommendation 55550 may include, for example, an indication of an optimized control loop 55544. In examples, ML model 55549 may receive the first and/or second control data, a tactical domain target 55546, historical data 55548, an indication of a portion of a procedure, and/or the like. In response to receiving data, ML model 55549 may generate a recommendation 55550 including a selection and/or indication of a control loop 55544 to be used by a first surgical element during a portion of a procedure.

At block 55588, the surgical computing system 55547 may transmit the recommendation 55550 to the first surgical element. In examples, a recommendation 55550 may cause the first surgical element to automatically select an optimized control loop 55544. Alternatively, the surgical computing system 55547 may provide a recommendation 55550 to an HCP 55508 (e.g., via a GUI). A recommendation 55550 provided to an HCP 55508 may include an indication to select an optimized control loop 55544 and/or an indication to configure a first surgical element based on a portion of a procedure. In examples, an HCP 55508 may determine whether to apply the recommendation 55550 to the first surgical element, to cause the first surgical element to select the optimized control loop 55544. Once the recommendation 55550 is transmitted to the first surgical element, the routine 55580 ends.

An optional routine may be performed by a surgical computing system 55547 to determine a recommendation 55550 for a portion of a procedure including one or more conflict zones 55572 of FIG. 8. In examples, a surgical computing system 55547 may receive a user input indicating a selection of a procedure from a plurality of procedures and/or an indication of a tactical domain target 55546 (e.g., similar to block 55581, and/or block 55584 of FIG. 9). The tactical domain target 55546 and/or the procedure may be associated with a physiological parameter of a patient 55541.

The surgical computing system 55547 may filter, based on the selection of the procedure, a plurality of surgical elements to obtain a primary surgical element 555543 and/or a secondary surgical element 55543n associated with the procedure (e.g., similar to block 55582 of FIG. 9). The primary surgical element 55543 may include a plurality of primary control loops associated with an output characteristic of the primary surgical element 55543, and the secondary surgical element 55543n may include a plurality of secondary control loops associated with an output characteristic of the secondary surgical element 55543n.

The surgical computing system 55547 may determine a tactical domain data (e.g., a tactical domain 55542) for the procedure (e.g., similar to block 55583 of FIG. 9). The tactical domain data may include one or more relationships associated with the primary surgical element 55543, the secondary surgical element 55543n, the parameter of the patient, and the tactical domain target 55546.

The surgical computing system 55547 may receive a primary control data 55545 from the primary surgical element 55543 based on a primary control loop 55544 from the plurality of primary control loops (e.g., similar to block 55586 of FIG. 9). The primary control data 55545 may include the output characteristic associated with the primary surgical element 55543.

The surgical computing system 55547 may receive a secondary control data 55545n from the secondary surgical element 55543n based on a secondary control loop 55544n from the plurality of secondary control loops (e.g., similar to block 55586 of FIG. 9). The secondary control data 55545n may include the output characteristic associated with the secondary surgical element 55543n.

The surgical computing system 55547 may determine conflict data (e.g., one or more conflict zones 55572) for the procedure. The conflict data may include a determination of a conflict associated with the primary surgical element 55543 and/or the secondary surgical element 55543n. The conflict data may include a request for a second user input indicating whether the determination of the conflict occurred during the procedure. The surgical computing system 55547 may generate a recommendation based on the conflict data (e.g., similar to block 55587 of FIG. 9). The surgical computing system 55547 may transmit the recommendation to the primary surgical element 55543 (e.g., similar to block 55588 of FIG. 9.). In examples, the recommendation to the primary surgical element 55543 may be the display 55570 of FIG. 8.

In examples, the surgical computing system 55547 may generate the recommendation further based on a machine learning (ML) model. The ML model may be trained using training data including one or more training data items. Each training data item of the one or more training data items may include at least one indication of the conflict data (e.g., conflict zones 55572).

The following is a non-exhaustive list of examples that may or may not be claimed:
1. A system for optimizing selection of a control loop for surgical elements during a medical procedure to achieve safe and reliable outcomes for patients, the system comprising a processor configured to:
   receive a user input indicating a selection of a procedure from a plurality of procedures, and a selection of a tactical domain target, wherein the procedure and the tactical domain target are associated with a parameter of a patient;
   filter, based on the selection of the procedure, a plurality of surgical elements to obtain a primary surgical element and a secondary surgical element associated with the procedure, wherein the primary surgical element comprises a plurality of primary control loops associated with an output characteristic of the primary surgical element, and the secondary surgical element comprises a plurality of secondary control loops associated with an output characteristic of the secondary surgical element;
   determine a tactical domain data for the procedure, wherein the tactical domain data comprises one or more relationships associated with the primary surgical element, the secondary surgical element, the parameter of the patient, and the tactical domain target;
   receive a primary control data from the primary surgical element based on a primary control loop from the plurality of primary control loops, wherein the primary control data comprises the output characteristic associated with the primary surgical element;
   receive a secondary control data from the secondary surgical element based on a secondary control loop from the plurality of secondary control loops, wherein the secondary control data comprises the output characteristic associated with the secondary surgical element;
   generate a recommendation based on the tactical domain data, the primary control data, and the secondary control data, wherein the recommendation comprises an indication of an optimized control loop for the primary surgical element during the procedure, wherein the optimized control loop adjusts the output characteristic associated with the primary surgical element to achieve the tactical domain target;
   send the recommendation to the primary surgical element; and
   cause the primary surgical element to adjust the output characteristic associated with the primary surgical element based on the optimized control loop, wherein the primary surgical element adjusts the output characteristic during the procedure to achieve the tactical domain target.
2. The system of example 1, wherein the parameter of the patient comprises at least one of oxygen saturation, blood pressure, respiratory rate, blood sugar, heart rate, a core body temperature and/or a hydration state.
3. The system of any of the preceding examples, wherein the tactical domain target is a core body temperature setpoint of the patient, the primary surgical element is a heating blanket, the secondary surgical element is a ventilator, the output characteristic associated with the primary surgical element is a heating coil of the heating blanket, the output characteristic associated with the secondary surgical element is a heating coil to adjust the temperature of air flowing through the ventilator, and wherein the recommendation comprises the indication of the optimized control loop to be used by the primary surgical element to control the heating coil of the heating blanket to meet the core body temperature setpoint.
4. The system of any of the preceding examples, wherein the processor is further configured to:
   obtain historical data associated with the procedure, wherein the historical data comprises historical control data for the primary surgical element and for the secondary surgical element; and
   generate the recommendation further based on a machine learning (ML) model, wherein the ML model is trained using training data comprising one or more training data items, wherein each training data item of the one or more training data items comprises at least one indication of the historical data associated with the procedure.
5. The system of any of the preceding examples, wherein the processor is further configured to:
   determine, for the procedure, conflict data, wherein the conflict data comprises:
      a determination of a conflict associated with the primary surgical element and the secondary surgical element; and
      a request for a second user input indicating whether the determination of the conflict occurred during the procedure; and
   generate the recommendation further based on a machine learning (ML) model, wherein the ML model is trained using training data comprising one or more training data items, wherein each training data item of the one or more training data items comprises at least one indication of the conflict data.
6. The system of any of the preceding examples, wherein the processor is further configured to:
   determine the tactical domain data further based on an ML model, wherein the ML model infers the one or more relationships associated with the primary surgical element, the secondary surgical element, the parameter of the patient, and the tactical domain target.
7. The system of any of the preceding examples, wherein the processor is further configured to:
   generate the recommendation based on an ML model associated with the tactical domain data, the primary control data, and the secondary control data.
8. The system of any of the preceding examples, wherein the one or more relationships associated with the primary surgical element, the secondary surgical element, the parameter of the patient, and the tactical domain target is determined based on a look-up-table.
9. A method for optimizing a selection of a control loop for surgical elements during a medical procedure to achieve safe and reliable outcomes for patients, the method comprising:
   receiving a user input indicating a selection of a procedure from a plurality of procedures, and a selection of a tactical domain target, wherein the procedure and the tactical domain target are associated with a parameter of a patient;
   filtering, based on the selection of the procedure, a plurality of surgical elements to obtain a primary surgical element and a secondary surgical element associated with the procedure, wherein the primary surgical element comprises a plurality of primary control loops associated with an output characteristic of the primary surgical element, and the secondary surgical element comprises a plurality of secondary control loops associated with an output characteristic of the secondary surgical element;
   determining a tactical domain data for the procedure, wherein the tactical domain data comprises one or more relationships associated with the primary surgical element, the secondary surgical element, the parameter of the patient, and the tactical domain target;
   receiving a primary control data from the primary surgical element based on a primary control loop from the plurality of primary control loops, wherein the primary control data comprises the output characteristic associated with the primary surgical element;
   receiving a secondary control data from the secondary surgical element based on a secondary control loop from the plurality of secondary control loops, wherein the secondary control data comprises the output characteristic associated with the secondary surgical element;
   generating a recommendation based on the tactical domain data, the primary control data, and the secondary control data, wherein the recommendation comprises an indication of an optimized control loop for the primary surgical element during the procedure, wherein the optimized control loop adjusts the output characteristic associated with the primary surgical element to achieve the tactical domain target;
   sending the recommendation to the primary surgical element; and
   causing the primary surgical element to adjust the output characteristic associated with the primary surgical element based on the optimized control loop, wherein the primary surgical element adjusts the output characteristic during the procedure to achieve the tactical domain target.
10. The method of example 9, wherein the parameter of the patient comprises at least one of oxygen saturation, blood pressure, respiratory rate, blood sugar, heart rate, a core body temperature and/or a hydration state.
11. The method of any of the preceding examples, wherein the tactical domain target is a core body temperature setpoint of the patient, the primary surgical element is a heating blanket, the secondary surgical element is a ventilator, the output characteristic associated with the primary surgical element is a heating coil of the heating blanket, the output characteristic associated with the secondary surgical element is a heating coil to adjust the temperature of air flowing through the ventilator, and wherein the recommendation comprises the indication of the optimized control loop to be used by the primary surgical element to control the heating coil of the heating blanket to meet the core body temperature setpoint.
12. The method of any of the preceding examples, further comprising:
   obtaining historical data associated with the procedure, wherein the historical data comprises historical control data for the primary surgical element and for the secondary surgical element; and
   generating the recommendation further based on a machine learning (ML) model, wherein the ML model is trained using training data comprising one or more training data items, wherein each training data item of the one or more training data items comprises at least one indication of the historical data associated with the procedure.
13. The method of any of the preceding examples, further comprising:
   determining, for the procedure, conflict data, wherein the conflict data comprises:
      a determination of a conflict associated with the primary surgical element and the secondary surgical element; and
      a request for a second user input indicating whether the determination of the conflict occurred during the procedure; and
   generating the recommendation further based on a machine learning (ML) model, wherein the ML model is trained using training data comprising one or more training data items, wherein each training data item of the one or more training data items comprises at least one indication of the conflict data.
14. The method of any of the preceding examples, further comprising:
   determining the tactical domain data further based on an ML model, wherein the ML model infers the one or more relationships associated with the primary surgical element, the secondary surgical element, the parameter of the patient, and the tactical domain target.
15. The method of any of the preceding examples, further comprising:
   generating the recommendation based on an ML model associated with the tactical domain data, the primary control data, and the secondary control data.
16. A system for optimizing selection of a control loop for surgical elements during a medical procedure to achieve safe and reliable outcomes for patients, the system comprising a processor configured to:
   receive a user input indicating a selection of a procedure from a plurality of procedures, and a selection of a tactical domain target, wherein the procedure and the tactical domain target are associated with a parameter of a patient;
   determine a tactical domain data for the procedure, wherein the tactical domain data comprises one or more relationships associated with a primary surgical element, a secondary surgical element, the parameter of the patient, and the tactical domain target;
   generate a recommendation based on the tactical domain data, wherein the recommendation comprises an indication of an optimized control loop for the primary surgical element, wherein the optimized control loop adjusts an output characteristic associated with the primary surgical element to achieve the tactical domain target; and
   send the recommendation to the primary surgical element.
17. The system of any of the preceding examples, wherein the parameter of the patient comprises at least one of oxygen saturation, blood pressure, respiratory rate, blood sugar, heart rate, a core body temperature and/or a hydration state.
18. The system of any of the preceding examples, wherein the tactical domain target is a core body temperature setpoint of the patient, the primary surgical element is a heating blanket, the secondary surgical element is a ventilator, and wherein the recommendation comprises the indication of the optimized control loop to be used by the primary surgical element to control a heating coil of the heating blanket to meet the core body temperature setpoint.
19. The system of any of the preceding examples, wherein the processor is further configured to:
   generate the recommendation further based on a machine learning (ML) model, wherein the ML model is trained using training data comprising one or more training data items, wherein each training data item of the one or more training data items comprises at least one indication of historical data associated with the procedure, and wherein the historical data comprises historical control data for the primary surgical element and for the secondary surgical element.
20. The system of any of the preceding examples, wherein the processor is further configured to:
   determine, for the procedure, conflict data comprising a determination of a conflict associated with the primary surgical element and the secondary surgical element; and
   generate the recommendation further based on the determined conflict data, wherein the recommendation comprises an indication of the conflict data.
21. A system for optimizing selection of a control loop for surgical elements during a medical procedure to achieve safe and reliable outcomes for patients, the system comprising a processor configured to:
   receive a user input indicating a selection of a procedure from a plurality of procedures, and a selection of a tactical domain target, wherein the procedure and the tactical domain target are associated with a parameter of a patient;
   filter, based on the selection of the procedure, a plurality of surgical elements to obtain a primary surgical element and a secondary surgical element associated with the procedure, wherein the primary surgical element comprises a plurality of primary control loops associated with an output characteristic of the primary surgical element, and the secondary surgical element comprises a plurality of secondary control loops associated with an output characteristic of the secondary surgical element;
   determine a tactical domain data for the procedure, wherein the tactical domain data comprises one or more relationships associated with the primary surgical element, the secondary surgical element, the parameter of the patient, and the tactical domain target;
   receive a primary control data from the primary surgical element based on a primary control loop from the plurality of primary control loops, wherein the primary control data comprises the output characteristic associated with the primary surgical element;
   receive a secondary control data from the secondary surgical element based on a secondary control loop from the plurality of secondary control loops, wherein the secondary control data comprises the output characteristic associated with the secondary surgical element;
   generate a recommendation based on the tactical domain data, the primary control data, and the secondary control data, wherein the recommendation comprises an indication of an optimized control loop for the primary surgical element during the procedure, wherein the optimized control loop adjusts the output characteristic associated with the primary surgical element to achieve the tactical domain target;
   determine, for the procedure, conflict data, wherein the conflict data comprises:
      a determination of a conflict associated with the primary surgical element and the secondary surgical element; and
      a request for a second user input indicating whether the determination of the conflict occurred during the procedure;
   generate the recommendation further based on a machine learning (ML) model, wherein the ML model is trained using training data comprising one or more training data items, wherein each training data item of the one or more training data items comprises at least one indication of the conflict data
   send the recommendation to the primary surgical element; and
   cause the primary surgical element to adjust the output characteristic associated with the primary surgical element based on the optimized control loop, wherein the primary surgical element adjusts the output characteristic during the procedure to achieve the tactical domain target

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A system for optimizing selection of a control loop for surgical elements, the system comprising a processor configured to:
receive a user input indicating a selection of a procedure from a plurality of procedures, and a selection of a tactical domain target, wherein the procedure and the tactical domain target are associated with a parameter of a patient;
determine a tactical domain data for the procedure, wherein the tactical domain data comprises one or more relationships associated with a primary surgical element, a secondary surgical element, the parameter of the patient, and the tactical domain target;
generate a recommendation based on the tactical domain data, wherein the recommendation comprises an indication of an optimized control loop for the primary surgical element, wherein the optimized control loop is configured to adjust an output characteristic associated with the primary surgical element to achieve the tactical domain target; and
send the recommendation to the primary surgical element.

2. The system of claim 1, wherein the parameter of the patient comprises at least one of oxygen saturation, blood pressure, respiratory rate, blood sugar, heart rate, a core body temperature and/or a hydration state,
and/or wherein the tactical domain target is a core body temperature setpoint of the patient, the primary surgical element is a heating blanket, the secondary surgical element is a ventilator, and wherein the recommendation comprises the indication of the optimized control loop to be used by the primary surgical element to control a heating coil of the heating blanket to meet the core body temperature setpoint.

3. The system of claim 1 or 2, wherein the processor is further configured to:
generate the recommendation further based on a machine learning, ,ML, model, wherein the ML model is trained using training data comprising one or more training data items, wherein each training data item of the one or more training data items comprises at least one indication of historical data associated with the procedure, and wherein the historical data comprises historical control data for the primary surgical element and for the secondary surgical element.

4. The system of any one of the preceding claims, wherein the processor is further configured to:
determine, for the procedure, conflict data comprising a determination of a conflict associated with the primary surgical element and the secondary surgical element; and
generate the recommendation further based on the determined conflict data, wherein the recommendation comprises an indication of the conflict data.

5. The system of any one of the preceding claims, wherein the processor is further configured to:
filter, based on the selection of the procedure, a plurality of surgical elements to obtain a primary surgical element and a secondary surgical element associated with the procedure, wherein the primary surgical element comprises a plurality of primary control loops associated with an output characteristic of the primary surgical element, and the secondary surgical element comprises a plurality of secondary control loops associated with an output characteristic of the secondary surgical element;
receive a primary control data from the primary surgical element based on a primary control loop from the plurality of primary control loops, wherein the primary control data comprises the output characteristic associated with the primary surgical element;
receive a secondary control data from the secondary surgical element based on a secondary control loop from the plurality of secondary control loops, wherein the secondary control data comprises the output characteristic associated with the secondary surgical element; and
cause the primary surgical element to adjust the output characteristic associated with the primary surgical element based on the optimized control loop, wherein the primary surgical element adjusts the output characteristic during the procedure to achieve the tactical domain target,
wherein generating the recommendation is further based on the primary control data and the secondary control data.

6. The system of claim 5, wherein the tactical domain target is a core body temperature setpoint of the patient, the primary surgical element is a heating blanket, the secondary surgical element is a ventilator, the output characteristic associated with the primary surgical element is a heating coil of the heating blanket, the output characteristic associated with the secondary surgical element is a heating coil to adjust the temperature of air flowing through the ventilator, and wherein the recommendation comprises the indication of the optimized control loop to be used by the primary surgical element to control the heating coil of the heating blanket to meet the core body temperature setpoint.

7. The system of claim 5 or claim 6, wherein the processor is further configured to:
obtain historical data associated with the procedure, wherein the historical data comprises historical control data for the primary surgical element and for the secondary surgical element; and
generate the recommendation further based on a machine learning, ML, model, wherein the ML model is trained using training data comprising one or more training data items, wherein each training data item of the one or more training data items comprises at least one indication of the historical data associated with the procedure.

8. The system of any one of claims 5 to 7, wherein the processor is further configured to:
determine, for the procedure, conflict data, wherein the conflict data comprises:
a determination of a conflict associated with the primary surgical element and the secondary surgical element; and
a request for a second user input indicating whether the determination of the conflict occurred during the procedure; and
generate the recommendation further based on a machine learning (ML) model, wherein the ML model is trained using training data comprising one or more training data items, wherein each training data item of the one or more training data items comprises at least one indication of the conflict data.

9. The system of any one of claims 5 to 8, wherein the processor is further configured to:
determine the tactical domain data further based on an ML model, wherein the ML model infers the one or more relationships associated with the primary surgical element, the secondary surgical element, the parameter of the patient, and the tactical domain target.

10. The system of any one of claims 5 to 9, wherein the processor is further configured to:
generate the recommendation based on an ML model associated with the tactical domain data, the primary control data, and the secondary control data.

11. The system of any one of claims 5 to 10, wherein the one or more relationships associated with the primary surgical element, the secondary surgical element, the parameter of the patient, and the tactical domain target is determined based on a look-up-table.

12. A method for optimizing a selection of a control loop for surgical elements performed by a processor of a system, the method comprising:
receiving a user input indicating a selection of a procedure from a plurality of procedures, and a selection of a tactical domain target, wherein the procedure and the tactical domain target are associated with a parameter of a patient;
filtering, based on the selection of the procedure, a plurality of surgical elements to obtain a primary surgical element and a secondary surgical element associated with the procedure, wherein the primary surgical element comprises a plurality of primary control loops associated with an output characteristic of the primary surgical element, and the secondary surgical element comprises a plurality of secondary control loops associated with an output characteristic of the secondary surgical element;
determining a tactical domain data for the procedure, wherein the tactical domain data comprises one or more relationships associated with the primary surgical element, the secondary surgical element, the parameter of the patient, and the tactical domain target;
receiving a primary control data from the primary surgical element based on a primary control loop from the plurality of primary control loops, wherein the primary control data comprises the output characteristic associated with the primary surgical element;
receiving a secondary control data from the secondary surgical element based on a secondary control loop from the plurality of secondary control loops, wherein the secondary control data comprises the output characteristic associated with the secondary surgical element;
generating a recommendation based on the tactical domain data, the primary control data, and the secondary control data, wherein the recommendation comprises an indication of an optimized control loop for the primary surgical element during the procedure, wherein the optimized control loop adjusts the output characteristic associated with the primary surgical element to achieve the tactical domain target;
sending the recommendation to the primary surgical element; and
causing the primary surgical element to adjust the output characteristic associated with the primary surgical element based on the optimized control loop, wherein the primary surgical element adjusts the output characteristic during the procedure to achieve the tactical domain target.

13. The method of claim 12, wherein the parameter of the patient comprises at least one of oxygen saturation, blood pressure, respiratory rate, blood sugar, heart rate, a core body temperature and/or a hydration state, and/or wherein the tactical domain target is a core body temperature setpoint of the patient, the primary surgical element is a heating blanket, the secondary surgical element is a ventilator, the output characteristic associated with the primary surgical element is a heating coil of the heating blanket, the output characteristic associated with the secondary surgical element is a heating coil to adjust the temperature of air flowing through the ventilator, and wherein the recommendation comprises the indication of the optimized control loop to be used by the primary surgical element to control the heating coil of the heating blanket to meet the core body temperature setpoint.

14. The method of claim 12 or claim 13, further comprising:
obtaining historical data associated with the procedure, wherein the historical data comprises historical control data for the primary surgical element and for the secondary surgical element; and
generating the recommendation further based on a machine learning (ML) model, wherein the ML model is trained using training data comprising one or more training data items, wherein each training data item of the one or more training data items comprises at least one indication of the historical data associated with the procedure.

15. The method of any one of claims 12 to 14, further comprising:
determining, for the procedure, conflict data, wherein the conflict data comprises:
a determination of a conflict associated with the primary surgical element and the secondary surgical element; and
a request for a second user input indicating whether the determination of the conflict occurred during the procedure; and
generating the recommendation further based on a machine learning (ML) model, wherein the ML model is trained using training data comprising one or more training data items, wherein each training data item of the one or more training data items comprises at least one indication of the conflict data.

16. The method of any one of claims 12 to 15, further comprising:
determining the tactical domain data further based on an ML model, wherein the ML model infers the one or more relationships associated with the primary surgical element, the secondary surgical element, the parameter of the patient, and the tactical domain target.

17. The method of any one of claims 12 to 16, further comprising:
generating the recommendation based on an ML model associated with the tactical domain data, the primary control data, and the secondary control data.

18. A computer-readable medium comprising instructions which, when executed by a processor, cause the processor to carry out the method of any one of claims 12 to 17.
